(19) Europäisches Patentamt
European Patent Office
Office européen des brevets

(11) **EP 3 513 836 B1**

(12) **EUROPÄISCHE PATENTSCHRIFT**

(45) Veröffentlichungstag und Bekanntmachung des Hinweises auf die Patenterteilung:
**25.11.2020 Patentblatt 2020/48**

(51) Int Cl.:
***A61N 2/02*** *(2006.01)*

(21) Anmeldenummer: **19152947.8**

(22) Anmeldetag: **22.01.2019**

(54) **MAGNETFELDAPPLIKATOR MIT EINEM RAMPENFÖRMIGEN SIGNALVERLAUF DER VERWENDETEN SPULENSTRÖME**

MAGNETIC FIELD APPLICATOR HAVING A RAMPED SIGNAL OF THE SOLENOID VALVE CURRENT USED

APPLICATEUR DE CHAMP MAGNÉTIQUE À UNE ALLURE DE SIGNAL DES COURANTS DE BOBINE UTILISÉS EN FORME DE RAMPE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.01.2018 DE 102018101394**

(43) Veröffentlichungstag der Anmeldung:
**24.07.2019 Patentblatt 2019/30**

(73) Patentinhaber: **Prof. Dr. Fischer AG**
**9492 Eschen (LI)**

(72) Erfinder: **FISCHER, Gerhard**
**9435 Heerbrugg (CH)**

(74) Vertreter: **Riebling, Peter**
**Patentanwalt**
**Postfach 31 60**
**88113 Lindau (DE)**

(56) Entgegenhaltungen:
**WO-A1-2004/087255 WO-A1-2016/074726**
**DE-U1-202016 008 331**

**Beschreibung**

Die Erfindung betrifft ein Behandlungsgerät mit einem Magnetfeldapplikator und einem rampenförmigen Signalverlauf der verwendeten Spulenströme nach dem Oberbegriff des Patentanspruchs 1. Die anderen Ausführungsbeispiele, Verfahren und Beispiele sind keine Teile der Erfindung.

Eine Vorrichtung mit dem Oberbegriff ist aus der eigenen EP 0 594 655 B1 bekannt geworden, die eine Vorrichtung betrifft, die mit niederfrequent gepulsten elektrischen Strömen arbeitet. Diese werden von einem Generator erzeugt, der mindestens eine daran angeschlossene Magnetfeldspule mit einem Spulenstrom versorgt. Die von der mindestens einen Magnetfeldspule erzeugten elektromagnetischen Felder dienen zur Beaufschlagung einer zu behandelnden Körperregion eines menschlichen oder tierischen Körpers.

Nachteil der EP 0 594 644 B1 ist, dass lediglich niedrige Feldstärken von bis zu 30 Mikrotesla erzeugt werden konnten und die verwendeten Spulen nicht in der Lage waren, stärkere Magnetfelder im Bereich von 1 Tesla oder mehr zu erzeugen.

Weiterer Nachteil war, dass die erzeugten Magnetfelder, die in der Art von Grundimpulsen in der Magnetfeldspule induziert wurden, von der behandelten Person als schmerzhaft und aggressiv empfunden wurden, was den Behandlungserfolg beeinträchtigte.

Bei einer Steigerung der Magnetfeldstärke kam es zu Schmerzempfindungen in der zu behandelnden Körperregion, was zum Abbruch der Behandlung zwang.

Wegen der Verwendung von schwachen Magnetfeldern kam es zu lange dauernden Behandlungen, was den therapeutischen Effekt beeinträchtigte.

Die genannte Druckschrift verwendete sogenannte Grundstromimpulse im Bereich zwischen 100 bis 1000 Hz, vorzugsweise 200 Hz, deren ansteigende Amplituden einer e-Funktion entsprachen. Den Grundstromimpulsen waren Hochfrequenzpulse mit einer Frequenz zwischen 10 und 100 kHz überlagert. Die Amplituden der daraus gebildeten Grundimpuls-Folgen wurden von einer periodischen, kurvenförmig an- und absteigenden Modulationsfrequenz im Bereich von 0,5 bis 35 Hz moduliert. Die so gebildeten Pulsfolgeserien waren deshalb im Ergebnis als sägezahnförmige Behandlungs-Stromimpulse ausgebildet.

Nachteil bei der Anwendung der sägezahnförmigen Behandlungs-Stromimpulse war allerdings die sich bei der Steigerung der Behandlungs-Stromimpulse einstellende Schmerzempfindung und damit eine Beeinträchtigung des Therapie-Erfolges.

Die WO 2004/087255 A1 offenbart ein Behandlungsgerät mit einem Magnetfeldapplikator gemäß dem Oberbegriff des Patentanspruchs 1.

WO 2016/074726 A1 offenbart ein Gerät zur Magnetfeldtherapie, bei dem die magnetische Feldstärke stufenförmig erhöht wird und dann bis zum Ende eines Behandlungszyklus auf einer bestimmten Feldstärke gehalten wird. Es werden keine Impulse mit unterschiedlicher Intensität verwendet.

DE 20 2016 008 331 U1 offenbart ein Vorrichtung mit Magnetfeldapplikator, bei der Impulse mit ansteigender Hüllkurve verwendet werden, deren Impulswiederholungsrate zwischen 0,01 bis 1000 Hertz liegt.

Der Erfindung liegt die Aufgabe zugrunde, eine Vorrichtung zur Magnetfeldtherapie der eingangs genannten Art so weiterzubilden, dass mit dem Magnetfeldapplikator verkürzte Behandlungszeiten bei höheren Spulenströmen mit einem verbesserten Therapieeffekt erzielt werden können.

Zur Lösung der gestellten Aufgabe ist die Erfindung durch die technische Lehre des Anspruches 1 gekennzeichnet.

Die Hüllkurve der Amplituden der Grundimpulse bildet einen in der Nähe von Null ausgehenden ansteigenden (rampenförmigen) Kurvenast aus, der etwa bis zur Mitte einer Behandlungszeit auf sein Maximum ansteigt und danach bis zum Ende der Behandlungszeit einen der maximalen Stromstärke entsprechend, gleichbleibenden Kurvenast ausbildet.

Beim Stand der Technik erreichte der genannte ansteigende, rampenförmige Kurvenast erst am Ende der Behandlungszeit sein Maximum der Stromstärke. Damit war der Nachteil verbunden, dass das Maximum der Stromstärke erst am Ende der Behandlungszeit erreicht wurde und dann abrupt abbrach. Damit wurde die Behandlung nicht mit der maximal möglichen Stromstärke durchgeführt. Die maximale Stromstärke des Spulenstroms konnte deshalb nicht wirksam werden.

Mit der technischen Lehre der Erfindung wird die maximale Stromstärke jedoch bereits in einem Bereich von 1/3 bis 2/3 der maximalen Behandlungszeit erreicht und kann dann bis zum Ende der Behandlungszeit wirken. Damit kann der maximale Spulenstrom wesentlich länger wirken. Eine solche Behandlungszeit kann etwa eine Dauer von 4 bis 10 Sekunden sein, an die sich eine Pause von z.B. 4 bis 10 Sekunden anschließt.

In einem bevorzugten Ausführungsbeispiel wird des bevorzugt, wenn die Hüllkurve etwa als ansteigende Gerade ausgebildet ist. Sie kann jedoch auch eine ansteigende Parabel-Kurve sein, eine ansteigende exponentielle Kurve oder eine andere ansteigende Kurve, deren Verlauf der Amplitude der verwendeten Grundimpulse entspricht.

Wichtig ist jedenfalls, dass vom Beginn des anfänglichen (startenden) Grundstromimpulses eine relativ geringe Stromamplitude gewählt wird, die dann entsprechend der Hüllkurve ansteigt, sodass damit eine Startrampe gebildet wird, die sich etwa über ein Drittel bis zu zwei Drittel der insgesamten Dauer einer Behandlungszeit mit diesen Grundimpulsen erstreckt und dann ein Maximum der Stromstärke erreicht, das bis zum Ende der Behandlungszeit beibehalten wird.

Die Offenbarung ist nicht darauf beschränkt, dass sich die Startrampe in einem Strom-Zeit-Diagramm über etwa die halbe Behandlungszeit erstreckt. Sie kann auch kürzer oder länger sein, und wichtig ist nur, dass die anfänglichen Grundimpulse und die daraus erzeugten Magnetfelder sehr langsam und einschleichend in das Gewebe eingetragen werden. Dadurch werden im Gewebe Spannungsimpulse evoziert, die erfindungsgemäß die damit gereizten Neuronen langsam auf größere Spannungsimpulse aufgrund größerer Magnetfeldstärken vorzubereiten, um so Schmerzempfindungen oder sogar Gewebeschädigungen zu vermeiden.

Mit dieser Art der einschleichenden Magnetfeldbehandlung hat sich herausgestellt, dass verbesserte Therapieerfolge erzielt werden können. Die maximale Stromstärke und das damit erzeugte maximale Magnetfeld konnte - im Gegensatz zum Stand der Technik - für eine längere Zeit aufrechterhalten werden.

Es konnte einerseits nachgewiesen werden, dass die Therapiezeit (insgesamt die Summe aller Behandlungszeiten) stark verkürzt werden konnte, und außerdem konnte nachgewiesen werden, dass auch der Therapieerfolg bei den hier verwendeten höheren Stromstärken im Vergleich zu den niedrigen Stromstärken bekannter Geräte gesteigert werden konnte.

In einer bevorzugten Ausführung entspricht die Hüllkurve deshalb einer Rechteck-oder e-Funktion oder Sägezahnfunktion.

Die Frequenz der Grundimpulse, die während einer Behandlungszeit (Haltezeit) erzeugt werden ist im Bereich zwischen 1 Hz bis 50 Hz.

Der Startwert des Erstimpulses der angewendeten Grundimpulse liegt im Bereich zwischen 1% bis 30 % der maximalen Amplitude. Die Amplitude des im ansteigenden Kurvenast erzeugten Grundimpulses entspricht etwa 90-100% der maximalen Amplitude, die dann in Form der maximalen Stromstärke im Bereich des gleichbleibenden Kurvenastes bis zum Ende der Behandlungszeit beibehalten wird. Die Behandlungszeit (Haltezeit) liegt im Bereich zwischen 1s bis 12s und die dazwischen liegende Pausenzeit liegt im Bereich zwischen 4s bis 12s.

Es wird demzufolge eine ansteigende Rampenkurve vorgeschlagen, die stets von einem bestimmten Anfangswert bis zu einem Endwert als ansteigende Kurve ausgebildet ist. Der Endwert eines Grundimpulses entspricht vorzugsweise einer Magnetfeldstärke von etwa 1,3 Tesla, und es wird bevorzugt bei einer Magnetfeldstärke von 0 beginnend mit der Rampenkurve eine allmähliche Steigerung der Grundimpulse erreicht, was bisher nicht erwartete therapeutische Effekte erzielt.

Durch die neuartige Magnetfeldsteuerung wird bei jeder eingestellten Maximalstromstärke immer von unten beginnend eine bestimmte Rampenfunktion (Hüllkurve der Amplituden der Grundimpulse) ausgeführt, solange bis die Rampenfunktion die eingestellte Maximalstromstärke erreicht hat und dann als gleichbleibender Kurvenast einer maximalen Stromstärke weiter geführt wird und dann abbricht.

Selbst wenn eine gleiche Magnetfeldstärke wie beim Stand der Technik verwendet wurde, hat sich herausgestellt, dass aufgrund der rampenförmigen Hüllkurve der Amplituden der Grundimpulse die bereits schon etwa in der Hälfte der Behandlungszeit ihr Maximum erreichten, bei einer verkürzten Behandlungszeit ein um 20 Prozent verbesserter Aufbau des behandelten Muskels erreicht werden konnte.

Außerdem sind die so erzeugten höheren Magnetfelder besonders stark fokussiert und haben eine deutlich höhere Angriffsfläche auf die behandelte Muskulatur, insbesondere gesamte Beckenboden-Muskulatur, was bisher - wegen der Verwendung von niedrigen Magnetfeldstärken - noch nicht bekannt war.

In einer Weiterbildung der Erfindung ist es vorgesehen, dass die aus den einzelnen Grundimpulsen gebildeten Behandlungsblöcke nunmehr ebenfalls in ihrer Amplitude einer bestimmten "Therapierampe" folgen, was bedeutet, dass auch die Amplituden der aus den einzelnen Grundimpulsen gebildeten Behandlungsblöcke einer bestimmten ansteigenden Kurve folgen, die als "Therapierampe" bezeichnet wird.

Diese Therapierampe (ansteigende Kurve) kann entweder als Gerade ausgebildet werden, als Parabelkurve oder als Exponentialkurve.

Bevorzugt ist auch hier, dass zum Beispiel die Behandlungsblöcke eine Behandlungsdauer von zum Beispiel 8 Sekunden aufweisen und aus einer Vielzahl von einzelnen Grundimpulsen bestehen, die jeweils einen Magnetfeldimpuls in der Magnetfeldspule induzieren.

Auch diese Behandlungsblöcke folgen demnach einer ansteigenden Therapierampe, und nach dem Ablaufen von in ihrer Amplitude periodisch ansteigenden Behandlungsblöcken von zum Beispiel acht Stück, wird dann die endgültige Maximalfeldstärke in der Magnetfeldspule erreicht.

Es wird demnach eine doppelte Rampenfunktion vorgeschlagen, nämlich einmal eine Startrampenfunktion in den einzelnen Behandlungsblöcken für die dort erzeugten Grundstromimpulse, und zum Zweiten zu einer Therapierampen-Funktion bei der Abfolge einer Vielzahl von aus einzelnen Grundimpulsen bestehenden Behandlungsblöcken.

In einer bevorzugten Ausgestaltung der Erfindung wird die erfindungsgemäße Stromsteuerung durch die Gate-Ansteuerung eines Thyristors ermöglicht, dem eine Freilaufdiode entgegengesetzt parallel geschaltet ist.

In einer Weiterbildung der vorliegenden Erfindung wird ein komplexes Therapiegerät verwendet, welches einen Zentralprozessor aufweist, der die gesamte Steuerung des Therapiegerätes übernimmt und in dem insbesondere die Größe des Amplitudenfensters, die Größe der Rampenfunktionen und dergleichen frei programmierbar sind.

In einer Weiterbildung der vorliegenden Erfindung ist es ferner vorgesehen, dass nunmehr die einzelnen Grundimpulse und die aus den einzelnen Grundimpulsen gebildeten Behandlungsblöcke einer bestimmten Behandlungsfrequenz folgen, die bevorzugt als Fibonacci-Frequenz bezeichnet wird.

Dies wird anhand der nachfolgenden Fibonacci-Skalierung mit dem Goldenen Schnitt als Grenzwert und deren Anwendung in Diagnostik und Therapie im Herz-Kreislauf-System des Menschen erläutert.

Der italienische Mathematiker Filius Bonacci (Fibonacci, 1170 - 1250) entdeckte diese Zahlenreihe, die mit ihrem Quotienten-Grenzwert zweier aufeinanderfolgender Zahlen zur berühmtesten Skalierung der Welt, zum "Goldenen Schnitt", führt. Viele Künstler, wie z. B. Leonardo da Vinci, wurden vom "Goldenen Schnitt" inspiriert. R. N. Elliot entwickelte in den 30er Jahren dieses Jahrhunderts auf diesen Grundlagen aufbauend eine Theorie, mit der man Trends- und Trendwenden in Natur und Technik, aber auch auf dem Aktienmarkt, aufzeigen konnte.

Die Fibonacci-Folge ist die unendliche Folge von natürlichen Zahlen, die (ursprünglich) mit zweimal der Zahl 1 beginnt oder (häufig, in moderner Schreibweise) zusätzlich mit einer führenden Zahl 0 versehen ist. Im Anschluss ergibt jeweils die Summe zweier aufeinanderfolgender Zahlen die unmittelbar danach folgende Zahl:

$$0, \ 1, \ 1, \ 2, \ 3, \ 5, \ 8, \ 13, \ ...$$

(optional) $0+1$ $1+1$ $1+2$ $2+3$ $3+5$ $5+8$

Die darin enthaltenen Zahlen heißen Fibonacci-Zahlen. Benannt ist die Folge nach Leonardo Fibonacci, der damit im Jahr 1202 das Wachstum einer Kaninchenpopulation beschrieb. Die Folge war aber schon in der Antike sowohl den Griechen als auch den Indern bekannt.

Weitere Untersuchungen zeigten, dass die Fibonacci-Folge auch noch zahlreiche andere Wachstumsvorgänge der Pflanzen beschreibt. Es scheint, als sei sie eine Art Wachstumsmuster in der Natur. Diese Erkenntnis macht sich die vorliegende Erfindung zunutze.

Die Fibonacci-Zahlen weisen einige bemerkenswerte mathematische Besonderheiten auf:

- Aufgrund der Beziehung zur vorherigen und zur folgenden Zahl scheint Wachstum in der Natur einem Additionsgesetz zu folgen.
- Die Fibonacci-Folge steht in einem unmittelbaren Zusammenhang zum Goldenen Schnitt. Je weiter man in der Folge fortschreitet, desto mehr nähert sich der Quotient aufeinanderfolgender Zahlen dem Goldenen Schnitt (1,618033...) an (beispielsweise 13:8=1,6250; 21:13≈1,6154; 34:21≈1,6190; 55:34≈1,6176; etc).
- Diese Annäherung ist alternierend, d. h. die Quotienten sind abwechselnd kleiner und größer als der Goldene Schnitt.

Problemstellung

Das komplexe System des menschlichen Blutkreislaufs, von Briggs und Peat /3/ so treffend als "erstaunliches Stück der Ingenieurskunst der Natur" bezeichnet, besteht bekanntlich aus einem "Versorgungssystem" (Hochdrucksystem), dem "Entsorgungssystem" (Niederdrucksystem) und der diese verbindenden Funktionseinheit "Mikrozirkulation" (Arteriolen, Kapillaren, Venolen), wobei das Herz mehr als eine "zentrale Pumpanlage" darstellt. Um das Kreislaufsystem in optimaler Zeit und auf kürzestem Weg beliebig nahe an jedem Körperteil zu bringen sowie das totale Blutvolumen niedrig zu halten, verzweigt sich die Blutversorgung bekanntlich zwischen 8 und 30 Mal, bevor sie jede Körperstelle erreicht /3/. Während dabei die Venen und Arterien mit ihren Verzweigungen in der Literatur oft als "chaotischer" Aufbau bezeichnet werden, soll im vorliegenden Beitrag gezeigt werden, dass im menschlichen Herz-Kreislauf-System eine Ordnungsform auftritt, der notwendigerweise Chaos zugrunde liegt: determiniertes Chaos. Hieraus lassen sich bei Annahme einer Fibonacci-Skalierung offensichtlich "Optimalwerte" für das gesamte Herz-Kreislauf-System ableiten, die die Grundlage für die Diagnostik, jedoch auch für eine Therapie (z. B. eine Magnetfeldtherapie nach der vorliegenden Erfindung), bilden sollen.

Es ist das Verdienst von Mandelbrot, West und Goldberger, erkannt zu haben, dass von einer "fraktalen Selbstähnlichkeit" auch im menschlichen Körper ausgegangen werden muss, dass sich das gleiche komplexe Verzweigungsmuster bei kleineren und immer kleineren Blutgefäßen wiederholt. "Es wurde mir immer klarer, dass Selbstähnlichkeit nicht etwa nur irgendeine uninteressante Eigenschaft ist, sondern ein mächtiges Mittel zum Hervorbringen von Gestalten" (Mandelbrot). Grundlage im Gefäßbaum ist dabei die Fibonacci-Skalierung mit ihrem Grenzwert 1,618034... während analog zur Lunge im Kapillarbereich zwecks Erreichung einer größeren Effizienz, zur Erzielung eines "Gleichgewichts zwischen physiologischer Ordnung und Chaos", von einer wesentlichen Skalenverschiebung ausgegangen werden muss.

Das Herz-Kreislauf-System des Menschen im Sinne der Selbstähnlichkeit betrachtet.

Ein Fundament der Selbstähnlichkeit bildet die berühmteste Skalierung der Welt: die Zahl 1,618034..., die aus der Fibonacci-Reihe

$$0, 1, 1, 2, 3, 5, 8, 13, 21, 34, 55,\ldots \qquad (1)$$

zunehmend als Quotienten-Grenzwert entsteht, wenn die jeweils nachfolgende durch die vorangehende Zahl der Reihe dividiert wird (Beispiel: 55/34=1,617647). Mit ihrem Kehrwert 0,618034... und ihrem Quadrat 2,618034... ist auch die Zahl 1,618034... selbstähnlich. Der Verhältnis-Grenzwert 1,618034 ist der bekannte Goldene Schnitt. Als Abkürzung wird nachfolgend 1,618 vereinbart.

Aufbauend auf Arbeiten von West und Goldberger zum "fraktalen Fibonacci-Lungenbaum" schlussfolgern Briggs und Peat, dass "der Körper eine Vernetzung von lauter selbstähnlichen Systemen wie den Lungen, den Gefäßsystemen, dem Nervensystem" ist. Es soll deshalb die These formuliert werden, dass die Struktur des aktiven Gefäßsystems im Optimum mit ausreichender Näherung nach einer Fibonacci-Skala modelliert werden kann und hierbei der irrationalen Zahl 1,618 ... einschließlich der Zusammenhänge

$$1,618^{-1} = 0,618 \quad 1,618^{1} = 1,618 \quad 0,5 \times 1,618^{0} = 0,5$$
$$1,618^{-2} = 0,382 \quad 1,618^{2} = 2,618 \quad 0,5 \times 1,618^{-1} = 0,309$$
$$1,618^{-3} = 0,236 \quad 1,618^{3} = 4,236 \quad 0,5 \times 1,618^{-2} = 0,191$$

im Sinne einer (Natur-) Konstanten eine dominierende Bedeutung zukommt. So ist vom "fraktalen Lungenbaum" bekannt, dass die Längenverhältnisse in den ersten sieben Generationen der Bronchialröhren in der menschlichen Lunge der Fibonacci-Skala folgen, die Durchmesser dieser Röhren sogar bis zu zehn Generationen. Danach tritt eine Änderung dieser Skalierung auf, um eine größere Effizienz in der Lunge zu erzielen.

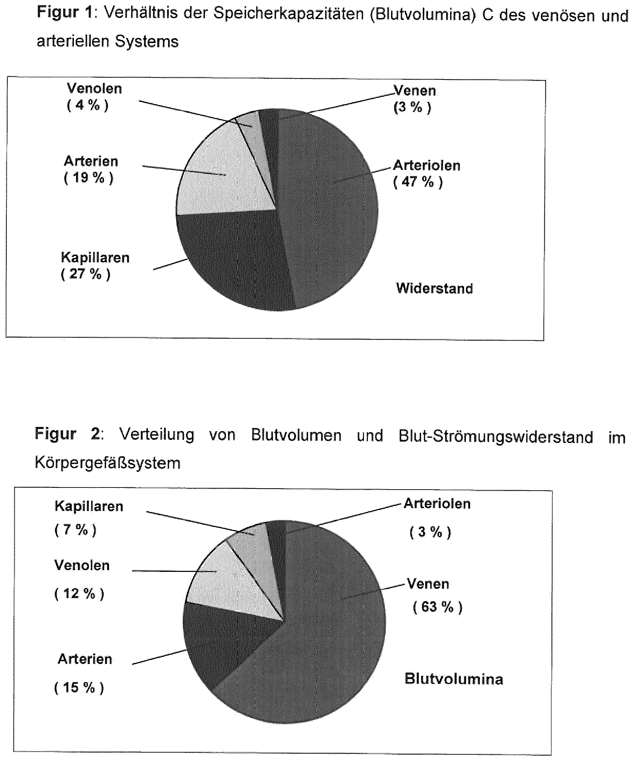

Die allgemeine Verteilung von Blutvolumen und Blut-Strömungswiderstand im Körpergefäßsystem ist in Figur 1 dargestellt.

Aus Figur 1 lassen sich u.a. folgende Relationen ableiten:

Das Verhältnis der Speicherkapazitäten (Blutvolumina) C des venösen und arteriellen Systems beträgt:

$$\frac{C_{ven\ddot{o}s}}{C_{arteriell}} = \frac{63\% + 12\%}{15\% + 3\%} = \frac{75\%}{18\%} = 4,17 \approx 4,236 = (1,618)^{3}. \qquad (2)$$

Offensichtlich sind die venösen und arteriellen Kapazitäten über die Verhältniszahl $4,236 = (1,618)^{3}$ optimiert.

Für das Verhältnis von Blut-Strömungswiderstand R und Blutvolumen C des kapillär-venolären Systems erhält man

$$\frac{R_{kapill\ddot{a}r,venol\ddot{a}r}}{C_{kapill\ddot{a}r,venol\ddot{a}r}} = \frac{31\%}{19\%} = 1,63 \approx 1,618. \qquad (3)$$

Für das kapilläre Widerstands-/Volumenverhältnis folgt aus Figur 2:

$$\frac{R_{kapill\ddot{a}r}}{C_{kapill\ddot{a}r}} = \frac{27\%}{6\ldots7\%} = 4,154 \approx 4,236 = (1,618)^{3}. \qquad (4)$$

Mit 21 Volumenprozenten als maximale O2-Sättigung des arteriellen Blutes und ca. 13 Volumenprozenten 02 im venösen Blut erhält man für deren Verhältnis und damit für die optimale Sauerstoffausschöpfung in den Kapillaren durch das

$$\frac{21\,Vol.\%\,O_2}{13\,Vol.\%\,O_2} = 1,615 \approx 1,618. \tag{5}$$

Gewebe.

Die Optimierung der Sauerstoffentnahme durch das Gewebe über die Zahl 1,618 erscheint als geradezu exemplarisch für die auch funktionell selbstähnliche Strukturierung des Kreislauf-Systems, mehr noch: für die selbstähnliche Einheit von Struktur und Funktion des "kleinen Kreislaufs", wie der fraktale Fibonacci-Lungenbaum zeigt.

Als Herz-Kreislauf-Normalwerte, die bei einem (20- bis 30-jährigen) Normalprobanden auftreten, werden in der Literatur angegeben:

Herzfrequenz: 70 - 75 $min^{-1}$ ($\equiv$ 1,17 ... 1,25 Hz),
Atmungsfrequenz: 16 - 18 $min^{-1}$ ($\equiv$ 0,27 ... 0,30 Hz),
Traube-Hering-Wellen als rhythmische Änderungen des arteriellen Blutdruckes: ca. 7 $min^{-1}$ ($\equiv$ 0,1 Hz),
Blutdruckwerte: systolisch $P_S$ = 120 mmHg,
diastolisch $P_D$ = 75 mmHg.

Aus diesen Normalwerten sollen nachfolgende Relationen abgeleitet werden:
Von Hildebrandt wurde bereits 1960 erkannt, dass zwischen Puls- und Atemrhythmus "koordinative Beziehungen" mit einem Norm-Frequenz-Verhältnis von 4 : 1 bestehen und Abweichungen von der Norm nach beiden Richtungen hin bestimmten Funktionsabweichungen im Herz-Kreislauf-System zugeordnet sind. Aus den angegebenen Normalwerten folgt das Verhältnis:

$$\frac{Herzfrequenz}{Atmungsfrequenz} = \frac{1,17...1,25\,Hz}{0,27...0,30\,Hz} = 4,25\,(Mittelwert) \approx 4,236 = (1,618)^3. \tag{6}$$

Es wird ebenso darauf verwiesen, dass der Atemrhythmus des Menschen gleichfalls "koordinative Beziehungen" zum 10-Sekunden-Rhythmus des Blutdruckes, der Traube-Hering-Wellen, besitzt. Mit den Normalwerten erhält man

$$\frac{Atmungsfrequenz}{Blutdruckperiodik} = \frac{0,27...0,30\,Hz}{0,1\,Hz} = 2,85\,(Mittelwert) \approx 2,618 = (1,618)^2. \tag{7}$$

Falls dem Herz-Kreislauf-System eine 1,618-Skalierung zugrunde liegt und sich darauf aufbauende Relationen ergeben, so ist zu erwarten, dass sich im Optimum ein solches Verhältnis auch für die Blutdruckwerte ergibt, das $V_{PS,PD}$ genannt werden soll. Eine Abweichung dieses Verhältnisses von der Norm wird offensichtlich ebenso auf Funktionsabweichungen im Herz-Kreislauf-System zurückzuführen sein (z. B. bei peripherer arterieller Verschlusskrankheit oder bei Mikrozirkulationsstörungen). In einer nicht veröffentlichten Arbeit wird dieses Verhältnis "relativer peripherer Widerstand $RPW_1$" genannt:

$$V_{Ps,PD} = RPW_1 = \frac{P_S}{P_D} \quad bzw.\,(mit\,den\,Normalwerten)\ V_{PS,PD} = \frac{120mmHg}{75mmHg} = 1,6 \approx 1,618. \tag{8}$$

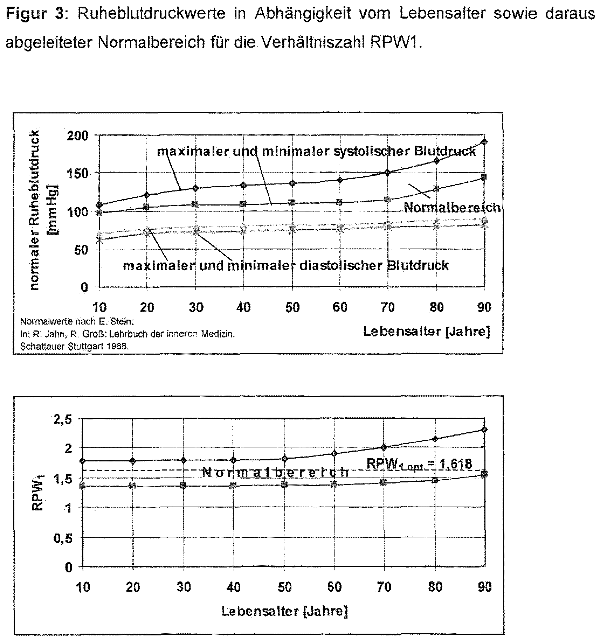

In Figur 3 sind Ruheblutdruck-Normalwerte und das daraus abgeleitete Verhältnis $RPW_1$ in Abhängigkeit vom Lebensalter dargestellt. Es ist ersichtlich, dass die irrationale Zahl 1,618 das Optimum ist, jedoch mit zunehmendem Lebensalter Abweichungen davon auftreten.

Bekannt ist des Weiteren, dass Häufigkeitsgipfel des Herzinfarktes, des plötzlichen Herztodes und des Schlaganfalls jeweils in den frühen Morgenstunden liegen. Es wurden deshalb 24h-Blutdruckmessungen sowohl bei normotonen als auch hypertonen Probanden durchgeführt.

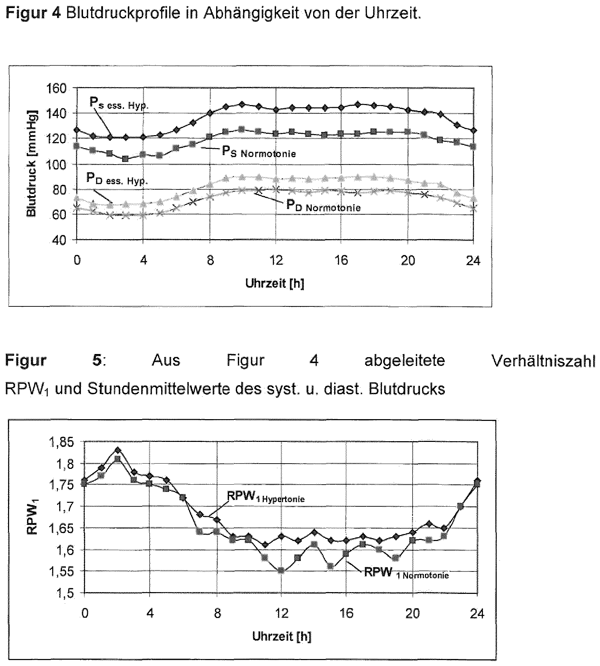

Dabei ergaben sich die in Figur 4 dargestellten Blutdruckprofile in Abhängigkeit von der Uhrzeit. Ermittelt man aus Figur 4 für beide Gruppen die Verhältniszahl $RPW_1$, so ergibt sich der Verlauf nach Figur 5. Figur 5 zeigt die Stundenmittelwerte des systolischen und diastolischen Blutdruckes bei Normotonie mit n = 111 und bei essentieller Hypertonie mit n = 109 Versuchspersonen und daraus abgeleitetes Verhältnis $RPW_1$.

Es ist ersichtlich:

- Die Theorie des optimalen Wertes bei der Zahl 1,618 wird bestätigt.

- Zwischen 8 und 20 Uhr erhält man sowohl bei normotonen als auch hypertonen Personen fast ideale RPW$_1$-Werte.
- Zwischen 1 und 3 Uhr wird dieses Verhältnis maximal. Erhöht es sich bei Patienten mit essentieller Hypertonie weiter, so ist ein Ernstfall vielleicht nicht auszuschließen.

Nachfolgend soll gezeigt werden, dass mit der Fibonacci-Skalierung ohne Vorgabe von Herz-Kreislauf-Normalwerten sowohl das (theoretische) Optimum anatomischer Verzweigungen des Gefäßsystems ermittelbar ist, als auch funktionelle Optima des Herz-Kreislauf-Systems einschließlich des EEG-Frequenzbereiches bestimmbar sind.

Selbstähnliche Systeme sind in der Literatur umfassend beschrieben. Ein solches Verhalten zeigen z. B. Baumstruktur, Flussverlauf, Momentaufnahme eines Blitzes, Verzweigung von Nervenzellen (Neuronen) sowie ein (arterieller) BlutGefäßbaum. Wird für deren Anzahl von Verzweigungen in den entsprechenden "Ebenen" dieser Systeme eine Fibonacci-Reihe nach Gl. (1) (als reduzierte Reihe) zugrunde gelegt, so würde man erhalten:

**1, 2, 3, 5, 8, 13, 21, 34, 55** .......

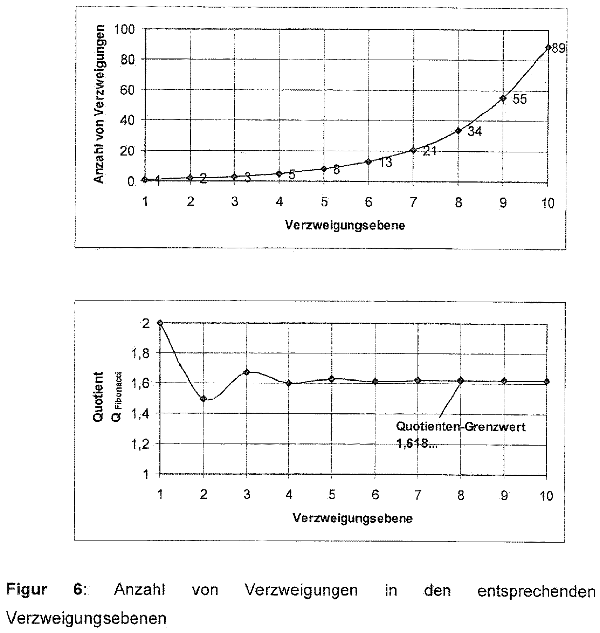

Als Quotient zweier aufeinanderfolgender Zahlen dieser Reihe stellt sich der Verlauf nach Figur 6 dar, wenn gebildet wird

$$Quotient_{Fibonacci} \ = \ Q_{Fibonacci} \ = \ \frac{x_n}{x_{n-1}} \tag{9}$$

und $x_n$ sowie $x_{n-1}$ die jeweils letzte bzw. vorletzte Fibonacci-Zahl der Reihe darstellen. Aus Figur 6 geht hervor, dass ab der 6. Verzweigungsebene der "Baumstruktur" die Abweichung vom Grenzwert 1,618... vernachlässigbar klein ist. Deshalb wird der Quotienten-Grenzwert 1,618..., der Goldene Schnitt, in der Literatur als "Skalierungsfaktor" bezeichnet.

Figur 6 zeigt die Anzahl von Verzweigungen in den entsprechenden Verzweigungsebenen sowie den Verlauf des Quotienten $Q_{Fibonacci}$ einschließlich sich einstellender Grenzwert 1,618..., falls eine Fibonacci-Reihe für die selbstähnlichen Systeme (z. B. Baumstruktur, Flussverlauf, Momentaufnahme eines Blitzes, Blutgefäßbaum, Verzweigung von Nervenzellen) zugrunde gelegt wird.

Nach der Literatur beträgt bei Normalprobanden der arithmetische Mittelwert der Querschnittsfläche der menschlichen Aorta als "Stamm" des arteriellen Blutgefäßbaumes $F_{Aorta} = \pi$ (cm$^2$). Diese irrationale Zahl, die auch zur Berechnung der Fläche und des Umfanges eines Kreises sowie des Volumens einer Kugel erforderlich ist, ist bekanntlich nicht exakt bestimmbar. Es ist bei deren Berechnung keinerlei Ordnung vorhanden, jede neu hinzukommende Dezimalzahl ist völlig zufällig.

Als Modell wird nachfolgend angenommen, dass die nach Erkenntnissen von West und Goldberger notwendigerweise optimale Bifurkation für relativ große Strecken des Blutvolumenflusses nach einer Fibonacci-Reihe erfolgt, indem die Aorta sich nach dieser Skalierung nicht nur verzweigt, sondern auch die entsprechenden Blutvolumenfluss-Geschwindigkeiten sich danach einstellen. Somit müsste es möglich sein, hierfür Optima für Herz- und Atemfrequenz sowie für weitere Herz-Kreislauf-Parameter abzuleiten.

Wird die nach Burton mittlere Fläche $\pi$ des Aortenquerschnittes formal mehrfach mit dem Fibonacci-Quotienten-Grenzwert 1,618 nach der Beziehung

$$F_n = \frac{\pi}{(1,618)^n} \tag{10}$$

skaliert (dividiert), so erhält man die überraschenden Resultate nach Tabelle 1, wobei n "Skalierungsgrad" genannt werden soll. Die daraus folgenden Faktoren $F_1$ ... $F_n$ entsprechen charakteristischen Bifurkationspunkten, offensichtlich gleichsetzbar mit im Herz-Kreislauf-System auftretenden "optimalen" Frequenzen. Während die in Tabelle 1 berechneten ("fett" markierten) Frequenz-Optimalwerte für das Herz-Kreislauf-System einschließlich EEG-Bereich sehr gut mit den Literaturangaben übereinstimmen, sind in dieser Tabelle auch "Zwischenfrequenzen" ersichtlich. Wenngleich diese Werte noch genauer zu überprüfen sind, zeigen die klinischen Erfahrungen bei den Atmungsfrequenzen unter Ruhebedingungen die Richtigkeit des Bereiches 0,175 ... 0,283 ... 0,458 Hz ($\equiv$ 10,5 ... 17 ... 27,5 min$^{-1}$).

Gleiches gilt auch für die Herzfrequenz-Bereiche 0,742 ... 1,2 ... 1,942 Hz ($\equiv$ 44,5 ... 72 ... 116,5 min$^{-1}$). Da jedoch auch höhere Herzfrequenzen als 116,5 min$^{-1}$ auftreten, insbesondere bei jüngeren Personen, ist dies offenbar nur dann möglich, wenn die Blutviskosität Normalwerte aufweist und nicht erhöht ist. Andererseits liegt bei anormal erhöhten Herzfrequenzen vielleicht eine "Störung" der vegetativen Komponente vor.

Mittels "dynamischer Systemdiagnostik" wurden sogenannte "arterielle und venöse Einschwingzeiten" zur Quantifizierung des hämodynamischen Zustandes von Extremitäten ermittelt und die gemessenen Normalwerte verglichen mit den Werten, wie sie durch obige Skalierung als "Optimalwerte" ableitbar sind. Die Übereinstimmung ist relativ gut, wobei sich derartige Optima bei den Skalierungsgraden n = 6, 7, 8 und 9 einstellten.

$$\text{Fibonacci-Skalierungs-Grundgleichung } F_n = \frac{\pi}{(1{,}618)^n}$$

**Tabelle 1:** Durch Mehrfachskalierung der mittleren Aortenfläche $F_{Aorta} = \pi$ [cm$^2$] abgeleitete zu erwartende optimale Herz-Kreislauf-Parameter einschließlich EEG-Bereich.

| n | optimale Frequenz $F_n$ [Hz] | Bedeutung |
|---|---|---|
| 7 | **0,108 ($\equiv$ 6,5 min$^{-1}$)** | **Blutdruckperiodik** (*Traube-Hering-Wellen*) / |
| 6 | 0,175 | untere Grenze Atmungsfrequenz (?) |
| 5 | **0,283 ($\equiv$ 17 min$^{-1}$)** | **Optimum Atmungsfrequenz** |
| 4 | 0,458 | obere Grenze Atmungsfrequenz (?) |
| 3 | 0,742 | untere Grenze Herzfrequenz (Bradykardie) ) |
| 2 | **1,2 ($\equiv$ 72 min$^{-1}$)** | **optimale Herzfrequenz/** |
| 1 | 1,942 | Herzeigenfrequenz (ungedämpft) |
| | | obere Grenze Herzfrequenz (Tachykardie) |
| 0 | **3,142** | **Optimum EEG-Delta-Bereich** |
| -1 | **5,08** | **Optimum EEG-Theta-Bereich** |
| -2 | **8,225** | **Beginn EEG-Alpha-Bereich,** |
| | | 1. Schumann-Resonanz / |
| -3 | **13,31** | **Ende EEG-Alpha-Bereich, Beginn Beta-Bereich** |
| | | (Beta $_{1,2}$) 2. Schumann-Resonanz |
| -4 | **21,53** | **EEG-Beta-Bereich** (Beginn Beta $_3$), |
| | | 3. Schumann-Resonanz |
| -5 | **34,84** | **Ende EEG-Beta-Bereich** |

Das zugrunde gelegte Modell und die vorgenommenen Skalierungen bedürfen weiterer Betrachtungen. Insbesondere ist zu analysieren, warum die hier formal abgeleiteten (eigentlich statischen) Faktoren $F_1 \ldots F_n$ offensichtlich mit (optimalen) Frequenzen gleichsetzbar sind, wie der Vergleich mit den Literaturangaben zeigt. Ausgehend vom Herzzeitvolumen, das als Blutvolumenstromstärke je Sekunde die Aorta mit seiner Fläche $F_{Aorta}$ passiert, sind hierfür die Gesetzmäßigkeiten der Strömung im Gefäßsystem für den Fall der Fibonacci-Skalierung anzuwenden.]

Wie in Tabelle 1 ersichtlich ist, erhält man für n = 0 bis -5 die optimalen Frequenzen **3**,142**; 5**,08**; 8**,225**; 13**,31; **21**,53 und **34**,84 Hz. Daraus geht hervor, dass die Zahlen **vor** dem Komma sämtlich Fibonacci-Zahlen darstellen, deshalb wurden sie "fett" markiert. Würde man die Frequenz bei n = -6 berechnen, so erhält man 56,37 Hz. Die Zahl 56 ist jedoch keine Fibonacci-Zahl, sodass diese Frequenz als nicht relevant für den EEG-Bereich betrachtet wird. Bekanntlich sind 35 Hz etwa die obere EEG-Frequenz-Grenze (Ende EEG-Beta-Bereich), wie sie aus experimentellen Messungen bestimmt wurde.

Aus Tabelle 1 folgen die bei einer 1,618-Skalierung von $\pi$ auftretenden "Optimalwerte" im Herz-Kreislauf-System:
Herzfrequenz $f_H$ = 1,2 Hz ($\equiv$ 72 min$^{-1}$),

Atmungsfrequenz $f_A$ = 0,283 Hz ($\equiv$ 17 min$^{-1}$), $\hfill$ (11)

Blutdruckperiodik $f_B$ = 0,108 Hz ($\equiv$ 6,5 min$^{-1}$).

Damit erhält man als **Relationen:**

$$\frac{f_H}{f_A} = \frac{1,2\ Hz}{0,283\ Hz} = 4,24 = (1,618)^3, \tag{12a}$$

$$\frac{f_A}{f_B} = \frac{0,283\ Hz}{0,108\ Hz} = 2,62 = (1,618)^2. \tag{12b}$$

Diese Optimalwerte einschließlich Relationen nach den Gln. (11) und (12) stimmen sehr gut mit den Normalwerten sowie deren Relationen überein.

Die in Tabelle 1 aufgeführten EEG-Frequenzen und deren Bedeutung für die erfindungsgemäße Magnetfeld-Therapie werden später erläutert.

Es wird deutlich, dass Periodenverdopplungen (bzw. Halbierungen) in Tabelle 1 durch eine Skalierung mit 1,618 nicht auf treten, auch nicht zwischen der Zahl $\pi$ = 3,1416... und der Skalierungskonstante 1,618... direkt. Hier wird lediglich ein Wert von 1,942 erreicht. Periodenverdopplung im Sinne der Entdeckung von Feigenbaum im Jahre 1976 führt bekanntlich zum "Aufwallen von Chaos", zum Anwachsen der Turbulenz und ist auf alle Fälle aus Stabilitätsgründen im Herz-Kreislauf-System zu verhindern. So ist auch bekannt, dass Herzversagen durch Stauung eintreten kann, falls Herzschlag und Atemrhythmus "allzu periodisch" werden. Dann liegt eine extreme Störung der normalen fraktalen Verhältnisse vor.

Vergleich von im Abschnitt 2. abgeleiteten (ausgewählten) Optimalwerten mit Messungen des natürlichen elektrischen und magnetischen Feldes

Der Raum zwischen Erde und Ionosphäre für die von Blitzen erzeugten Signale (Atmospherics) stellt einen sogenannten Wellenleiter dar, in dem sich diese Signale entsprechend ihrer Frequenz und dem Zustand der Ionosphäre mehr oder weniger stark gedämpft als elektromagnetische Wellen ausbreiten.

Die meisten zumindest der bei schönem Wetter über der Erdoberfläche gemessenen Vorgänge des elektrischen und magnetischen Feldes, die eine Frequenz von einigen Hertz haben, resultieren aus der Anregung des Erdelonosphären-Hohlraumresonators durch weit entfernte Gewitter. Diese Erscheinung ist in der Physik als "Schumann-Resonanz" bekannt. Sie reicht von etwa 7,8 Hz bis ca. 35 Hz und ist damit frequenzmäßig identisch mit dem gesamten Alpha- und Beta-Bereich im Elektroenzephalogramm (EEG). Vergleicht man die Registrierungen der Schumann-Resonanz-Signale mit denen des EEG, so ist es meist schwierig zu unterscheiden, ob es sich um eine Aufzeichnung von Gehirnströmen oder von in der Natur vorkommenden elektrischen Feldschwankungen handelt. In [Toomey, J. and C. Polk: Research on Extremely Low Frequency Propagation with Particular Emphasis on Schumann Resonance and Related Phenomena. University of Rhode Island, Kingston, R. I., USA. Contract No. AF 19 (628) - 4950. 1. April 1970] ist das Frequenzspektrum eines gemessenen vertikalen elektrischen Feldes natürlicher Signale wiedergegeben.

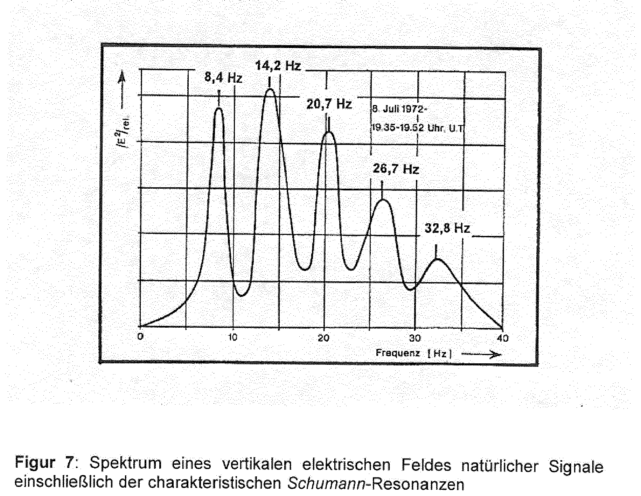

Figur 7 zeigt diese Messungen, wo deutlich die Schumann-Resonanzen als typische Resonanzerscheinungen ersichtlich sind. Setzt man diese Werte in Relation zu denen, die mittels Fibonacci-Skalierung gefunden wurden (s. Tabelle 1), so zeigt sich eine sehr gute Übereinstimmung. Die Fibonacci-Skalierungs-Frequenzen können jedoch nur verglichen werden mit den Resonanzfrequenzen des ungedämpften "Erde-Ionosphären-Hohlraumresonators" (D=0). Eine solche Dämpfung D = 0 ist aber in der Natur ausgeschlossen, sodass sich Abweichungen vor allem bei den höheren Schumann-Resonanzen, also bei Frequenzen oberhalb von 22 Hz, ergeben, die ersten 3 Frequenzmaxima sind nahezu identisch.

Die "Resonanzgüte" ist vor allem bei den höheren Frequenzen relativ klein:

| **Schumann-Resonanz:** | 8,4 | 14,2 | 20,7 | Hz |
|---|---|---|---|---|
| ↕ | ↕ | ↕ |
| ***Fibonacci*-Frequenzskalierung:** | 8,225 | 13,31 | 21,53 | Hz |

Da diese Frequenzen zugleich typische EEG-Frequenzen darstellen (Beginn und Ende Alpha-Bereich sowie Beta-Bereich) und letztere auch bei der Wirkung von Pharmaka entscheidend sind, lassen sich aus diesen Ergebnissen wichtige Schlussfolgerungen für Therapie-Verfahren (einschließlich der erfindungsgemäßen Magnetfelder ableiten.

Weiterhin ist aus Erdmagnetfeldmessungen bekannt, dass Schwingungen mit einer Variationsbreite der Periodendauern von 30 Sekunden ($\equiv$ 0,033 Hz) bis 0,025 Sekunden ($\equiv$ 40 Hz) auftreten. Damit ist diese Bandbreite mit der des elektrischen Feldes und des EEGs sowie mit der Fibonacci-Skalierung 34,84 Hz identisch, sodass von einer offensichtlichen Redundanz des elektrischen und magnetischen Feldes ausgegangen werden muss. Auffallend bei den Erdmagnetfeldmessungen waren jedoch Schwingungen mit der Periodendauer von 4,5 Sekunden ($\equiv$ 0,22 Hz), wenngleich sie nur mit einer Intensität von $10^{-12}$ Tesla auftraten. Diese Komponente entspricht, wie vorher gezeigt wurde, offensichtlich der Atmungsfrequenz. Folglich kann daraus geschlussfolgert werden, dass das menschliche Herz-Kreislauf-System bei der Atmung "unterstützt" wird durch das Erdmagnetfeld. Der ermittelte Frequenzwert unterscheidet sich wenig vom ermittelten Optimum $f_A = 0,283$ Hz auf der Basis der Fibonacci-Skalierung, also kann auch hier von einer sehr guten Übereinstimmung von Modell und Wirklichkeit ausgegangen werden.

Dass das Magnetfeld der Erde allgemein zeitlichen Schwankungen unterworfen ist und aus solchen Messwerterfassungen auch die quantitativen Schumann-Resonanzen ableitbar sind, ist bekannt.

Zusammenfassung

Mithilfe der von Mandelbrot, West und Goldberger eingeführten "fraktalen Selbstähnlichkeit" wird gezeigt, dass die Fibonacci-Skalierung mit ihrem Grenzwert 1,618034... (Goldener Schnitt) ein Strukturmodell zur Quantifizierung von Kenngrößen und Parametern im Herz-Kreislauf-System des Menschen darstellt. Legt man für eine optimale Leistungs- bzw. Energieübertragung vom Herzen bis in die Gefäßperipherie eine solche Fibonacci-Gefäßstruktur zugrunde, so lassen sich hierfür Optimalwerte der Herzperiodendauer / Herzfrequenz, Atmung sowie der Blutdruckperiodik bestimmen, jedoch auch für den EEG-Bereich als Therapie-Frequenzen für angewendete Magnetfelder. In diesem Sinne optimiert sich auch die Sauerstoffentnahme durch das Gewebe. Gleichfalls erhält man auf der Basis des Grenzwertes 1,618034 charakteristische Relationen, die sich zwischen den Kenngrößen und Parametern einstellen.

Die abgeleiteten Optimalwerte einschließlich Relationen stimmen sehr gut mit den bekannten klinischen Normalwerten überein.

Das Modell "Fibonacci-Skalierung mit Grenzwert 1,618034..." kann offensichtlich auch dem natürlichen elektromagnetischen Feld zugrunde gelegt werden. Die aus den praktischen Messungen des natürlichen elektrischen Feldes des Erdelonosphären-Hohlraumresonators gewonnenen Frequenzspektren, speziell die Schumann-Resonanzen, sind sehr gut vergleichbar mit den EEG-Frequenzbereichen sowie mit den Frequenzen, wie sie mittels Fibonacci-Skalierung erhalten werden. Das betrifft auch die Frequenzen und -bereiche, die aus Erdmagnetfeldmessungen abgeleitet wurden. Der Frequenzvergleich zwischen dem natürlichen elektrischen und dem Erdmagnetfeld macht zugleich eine Redundanz deutlich, die offensichtlich für die Entwicklung des Lebens auf unserer Erde erforderlich war und ist.

Somit wurde mit der vorstehenden Darlegung nachgewiesen, dass eine Verbesserung des Therapieeffektes durch die besondere Frequenz-Skalierung der angewendeten Magnetfelder erreicht werden kann, was bisher nicht bekannt war.

Der Magnetfeldapplikator ist Teil eines Behandlungsgeräts zur Magnetfeldtherapie. Das Behandlungsgerät kann in Form eines Behandlungsstuhls ausgebildet sein und einen oder mehrere Magnetfeldapplikatoren aufweisen. Jeder Magnetfeldapplikator umfasst mindestens eine Behandlungsspule mit einem Magnetkern, die in das Behandlungsgerät eingebaut sind und ein aus einer Ebene, z.B. Sitzfläche, Rückenlehne, Fußteil, des Behandlungsgeräts austretendes magnetisches Feld erzeugen.

Das aus der Ebene des Behandlungsgeräts austretende Magnetfeld weist ein räumliches Volumen auf und bildet eine Plateaufläche in x- und y-Richtung der Ebene des Behandlungsgeräts mit einer relativ homogenen magnetischen Flussdichte.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:

Figur 1: Verhältnis der Speicherkapazitäten (Blutvolumina) C des venösen und arteriellen Systems

Figur 2: Verteilung von Blutvolumen und Blut-Strömungswiderstand im Körpergefäßsystem

Figur 3: Ruheblutdruckwerte in Abhängigkeit vom Lebensalter sowie daraus abgeleiteter Normalbereich für die Verhältniszahl RPW1

Figur 4: Blutdruckprofile in Abhängigkeit von der Uhrzeit

Figur 5: Aus Figur 4 abgeleitete Verhältniszahl $RPW_1$ und Stundenmittelwerte des syst. u. diast. Blutdrucks

Figur 6: Anzahl von Verzweigungen in den entsprechenden Verzweigungsebenen

Figur 7: Spektrum eines vertikalen elektrischen Feldes natürlicher Signale einschließlich der charakteristischen Schumann-Resonanzen

Figur 8: schematisierte Darstellung eines bevorzugten Behandlungsgerätes

Figur 9: Darstellung des Spulenstroms nach dem Stand der Technik

Figur 10: die Darstellung der aus den einzelnen Spulenströmen gewonnenen Stromimpulse nach dem Stand der Technik

Figur 11: die Darstellung der aus den einzelnen Grundimpulsen gebildeten Behandlungsblöcke nach dem Stand der Technik

Figur 12: die Darstellung der Erfindung im Vergleich zur Figur 3 mit Angabe verschiedener Rampenfunktionen in einer ersten Ausführung

Figur 13: die gleiche Darstellung wie Figur 12 mit einer unterschiedlichen Rampenfunktion in einer zweiten Ausführung

Figur 14: die Darstellung der einzelnen Behandlungsblöcke nach der Erfindung mit jeweils Grundimpulsen, die einer Rampenfunktion folgen

Figur 15: eine weitere Ausgestaltung der Darstellung nach Figur 14, bei der auch die Behandlungsblöcke einer Rampenfunktion folgen

Figur 16: die Darstellung einer Thyristor/Dioden-Ansteuerung mit einer Schaltung nach Figur 17

Figur 17: die Darstellung der Thyristor/Dioden-Ansteuerung

Figur 18: die Tabelle, die zu den Zeichnungen nach Figuren 16 und 17 gehört

Figur 19: ein schematisiertes Blockschaltbild eines Behandlungsgerätes mit Darstellung der einzelnen Funktionsblöcke

Figur 20: die Darstellung gegenüber Figur 12 in abgewandelter Ausführungsform, bei der eine an- und absteigende Rampenfunktion als E-Funktion innerhalb einer Behandlungszeit gewählt ist

Figur 21: eine Abwandlung gegenüber Figur 20, bei der die Rampenfunktion allein einer in der Behandlungszeit ansteigenden E-Funktion entspricht

Figur 22: die Funktion einer Hüllkurve, wie sie später für die Formung der Stromimpulse verwendet wird

Figur 23: die Darstellung der Trigger-Impulse, die zwischen den Hüllkurven nach Figur 22 gesetzt sind

Figur 24: die Darstellung, wie eine Vielzahl von Grundimpulsen im Bereich der Hüllkurven untergebracht wird

Figur 25: die Darstellung, wie eine Vielzahl von eine Hüllkurve aufweisenden Behandlungsblöcken nach Figur 24 in einem bestimmten Frequenzmuster aufeinanderfolgen

Figur 26: die Darstellung, wie diese Frequenzmuster noch unter sich gruppiert werden, um eine überlagerte Frequenz zu erzeugen

Figur 27: eine Darstellung, wie die Fibonacci-Frequenzen verteilt sind

Figur 28: eine schematisierte Darstellung, wie die aus einzelnen Grundimpulsen gebildeten Behandlungsblöcke zu größeren Behandlungsblöcken zusammengesetzt werden, und diese wiederum zu weiteren Behandlungsblöcken, die dann einem bestimmten Frequenzmuster folgen.

Figur 29: eine Darstellung der Messpunktverteilung in der x-y Ebene

Figur 30: den gemessenen Spulenstrom bei den Intensitätseinstellungen 20%, 40%, 60%, 80% und 100%

Figur 31: Räumliche Verteilung der magnetischen Flussdichte in der Sitzebene (z = 0)

Figur 32: Räumliche Verteilung der magnetischen Flussdichte in der Beckenbodenebene (z = 12 cm)

Figur 33: Räumliche Verteilung der magnetischen Flussdichte in der Herzebene (z = 35 cm)

Figur 34: Darstellung der magnetischen Flussdichte (Zeitverlauf) in der Sitzebene (z = 0) entlang der positiven x-Achse an fünf Messstellen

Figur 35: Darstellung der magnetischen Flussdichte (Zeitverlauf) in der Beckenbodenebene (z = 12 cm) entlang der positiven x-Achse an fünf Messstellen

Figur 36: Darstellung der magnetischen Flussdichte (Zeitverlauf) in der Herzebene (z = 35 cm) entlang der positiven x-Achse an fünf Messstellen

Figur 37: Darstellung der magnetischen Flussdichte in der Sitzebene (z = 0) entlang der positiven x- Achse an fünf Messstellen bei verschiedenen Intensitätseinstellungen

Figur 38: Darstellung der magnetischen Flussdichte in der Beckenbodenebene (z = 12 cm) entlang der positiven x-Achse an fünf Messstellen bei verschiedenen Intensitätseinstellungen

Figur 39: Darstellung der magnetischen Flussdichte in der Herzebene (z = 35 cm) entlang der positiven x-Achse an fünf Messstellen bei verschiedenen Intensitätseinstellungen

Figur 40: schematisierte Darstellung einer abgewandelten Ausgestaltung eines bevorzugten Behandlungsgerätes.

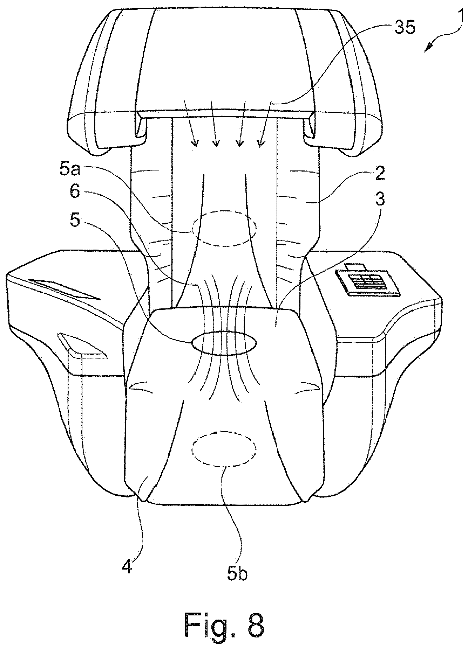

Das in Figur 8 dargestellte Behandlungsgerät 1 besteht in dem bevorzugten Ausführungsbeispiel aus einem Behandlungsstuhl, welcher insgesamt eine Rückenlehne 2, eine Sitzfläche 3 und ein Fußteil 4 aufweist.

Die Rückenlehne 2 ist von der Sitzfläche 3 nach hinten abklappbar, und das Fußteil 4 ist abklappbar oder hochklappbar, je nachdem, welche Körperteile auf dem Behandlungsgerät 1 behandelt werden sollen.

Die zu behandelnde Person sitzt auf dem Behandlungsgerät 1, welches als Sessel ausgebildet ist, und wird von der Kopfseite her mit zusätzlichen Lichtquellen bestrahlt, die von einem Applikator 35 in der Oberseite der Rückenlehne 2 erzeugt werden.

Die Darstellung in Figur 8 zeigt die Anordnung einer ersten möglichen Behandlungsspule 5, die beispielsweise in der Sitzfläche 3 angeordnet ist und ein geeignetes Magnetfeld 6 von zum Beispiel einer Maximalfeldstärke von 1,6 Tesla erzeugt, welches Magnetfeld 6 die zu behandelnden Körperteile der auf dem Behandlungsgerät 1 sitzenden Person durchströmt und entsprechende Elektropotenziale in den Muskelfasern auslöst, um so eine periodische oder aperiodische Muskelkontraktion zu erzeugen.

Die Muskelkontraktionen erfolgen durch die die Muskelfasern enervierende Nerven, die von einer durch das Wechselmagnetfeld erzeugten Reizspannung beaufschlagt sind. Es handelt sich um eine Magnetstimulation basierend auf dem Faradayschen Prinzip, dass ein veränderliches Magnetfeld (zeitlich oder räumlich) einen Stromfluss im Gewebe erzeugt. Im Rhythmus der Frequenzschaltung z.B. zwischen 10-50 Hz kommt es im effektiv wirkenden Feld zu einer Depolarisation von motorischen Nerven und Muskeln und konsekutiv zu einer starken Kontraktion der quergestreiften Muskulatur. Dies ist eine Folge der Freisetzung von Neurotransmittern an der motorischen Endplatte.

Als additives Wirkprinzip gilt die Stimulation afferenter Fasern der beaufschlagten Nerven.

In einer anderen Ausgestaltung des Behandlungsgerätes 1 kann es vorgesehen sein, dass zusätzlich oder in Alleinstellung eine zusätzliche Behandlungsspule 5a in der Rückenlehne 2 angeordnet ist, oder dass alternativ und optional auch eine zusätzliche oder allein eine weitere Behandlungsspule 5b im Fußteil 4 angeordnet ist.

Es kann auch vorgesehen sein, dass die dort gezeigten Behandlungsspulen 5, 5a, 5b in Längsrichtung des Behandlungsgerätes 1 einstellbar ausgebildet und verschiebbar sind und ein oder mehrere Behandlungsspulen können auch entfallen, sodass beispielsweise nur eine Behandlungsspule 5 im Sitzteil oder nur eine Behandlungsspule 5a in der Rückenlehne oder nur eine Behandlungsspule 5b im Fußteil vorhanden sind.

Es kann auch vorgesehen sein, dass eine oder mehrere der Behandlungsspulen 5, 5a, 5b als handhabbare Behandlungsspule verwendet werden können, das heißt, sie wird in der Hand gehalten und wird auf ein spezielles Körperteil der behandelnden Person aufgelegt. Solche Applikatoren sind in der EP 0 594 655 B1 gezeigt.

In der gezeigten Stellung ist das Behandlungsgerät 1 als Sessel ausgebildet, worauf die Erfindung nicht beschränkt ist. Das Behandlungsgerät 1 kann auch als Liege ausgebildet sein, als Hocker oder dergleichen mehr und kann ein oder mehrere Spulen tragen.

Es hat sich im Übrigen herausgestellt, dass eine zusätzliche Lichtbehandlung am Behandlungsgerät 1 mit einem geeigneten Applikator 35 sehr gut wirkt.

Solange die Magnetfelder 6 in einer der Behandlungsspulen 5, 5a, 5b wirken, wirkt auch das von dem Applikator 5 erzeugte Lichtfeld sehr viel stärker, und die Wirkung des Lichtfeldes steigert zusätzlich den Therapie-Erfolg.

Es hat sich herausgestellt, dass eine Lichteinwirkung von zum Beispiel 10.000 Lux für eine alleinige Lichttherapie zu einer Behandlungsdauer von mindestens einer Stunde führt. Versuche haben nun gezeigt, dass wenn die Lichttherapie mit der Magnetfeldtherapie mit Magnetfeldern 6 kombiniert wird, nun mehr nur noch Behandlungsdauern von 10 Minuten ausreichen, um den gleichen gewünschten Therapier-Erfolg zu erreichen.

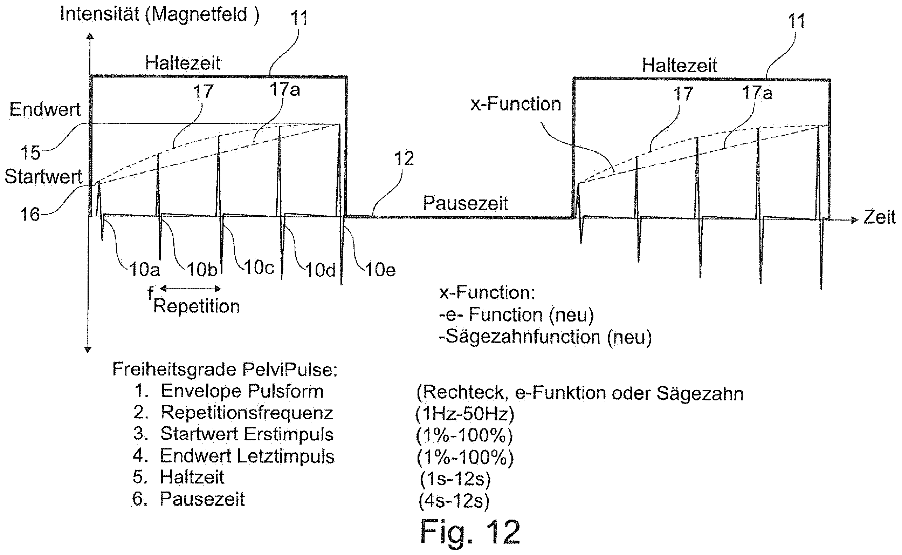

Versuche haben im Übrigen gezeigt, dass der besondere Effekt der positiven Einwirkung der Lichttherapie über den Applikator 35 nur dann zu einem Erfolg führt, wenn die verwendeten Magnetfelder einer Rampenfunktion folgen, wie anhand der Figuren 12, 13 und 14 erläutert wird.

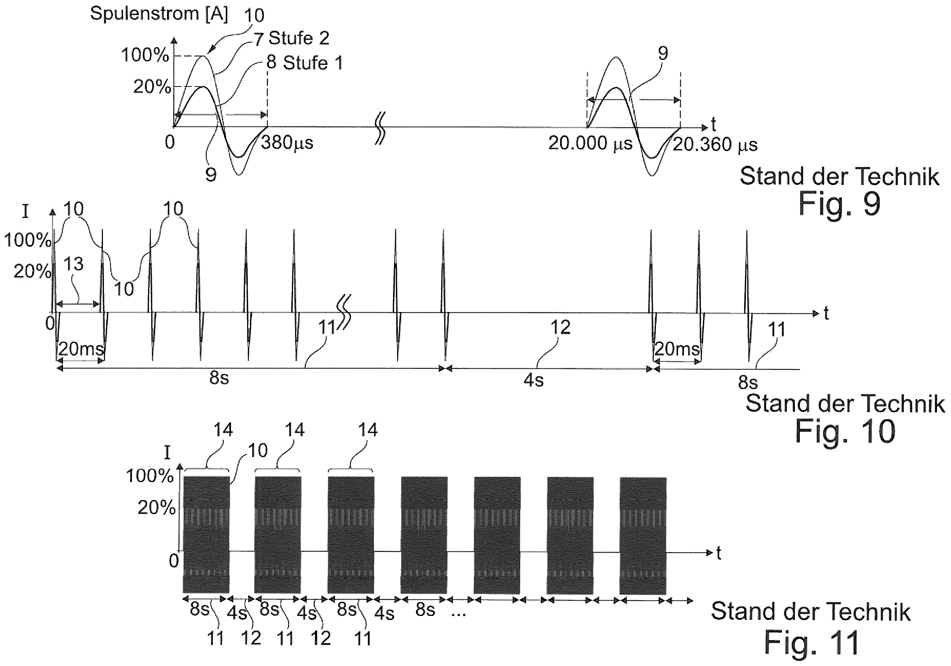

Anhand der Figuren 9 bis 11 wird zunächst die Grundfunktion zur Erzeugung der Magnetfelder 6 erläutert, wie sie beispielsweise aus der eigenen Patentschrift EP 0 594 655 B1 zu entnehmen ist.

In Figur 9 ist dargestellt, dass der Spulenstrom in zwei verschiedenen Stufen erzeugt werden kann, wie zum Beispiel in der Stufe 1 und Stufe 2.

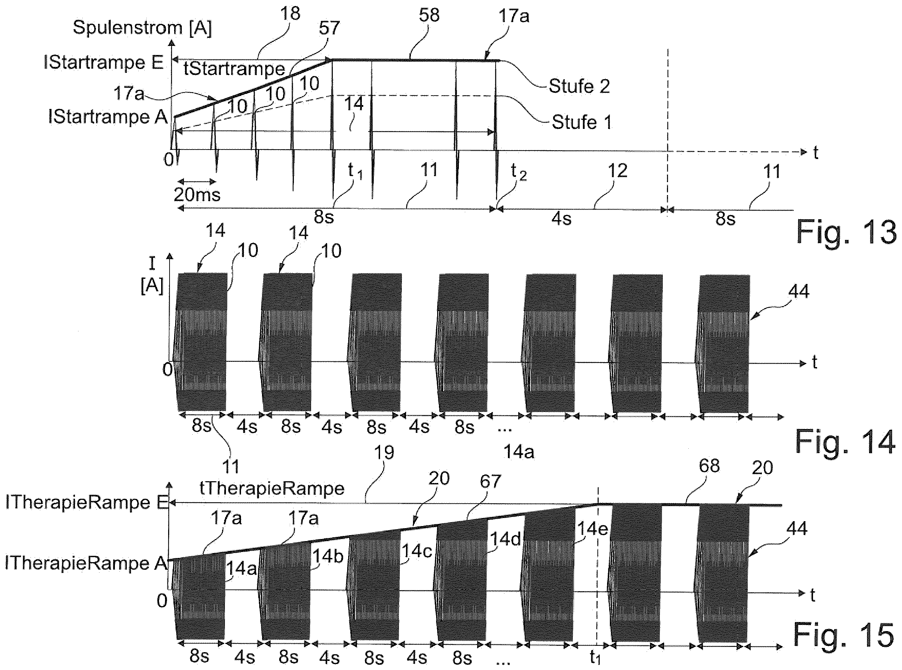

Die zwei unterschiedlichen Stufen entsprechen den am Steuergerät 55 einstellbaren zwei unterschiedlichen Stromstärken für zwei unterschiedlich starke Magnetfelder. Diese zwei unterschiedlichen Stromstärken sind auch in den Figuren 14 und 15 eingezeichnet.

Der Spulenstrom 8 in der Stufe 1 ist etwa nur 20 Prozent des maximal erreichbaren Spulenstroms, während der Spulenstrom 7 dem Spulenstrom von 100 Prozent entspricht.

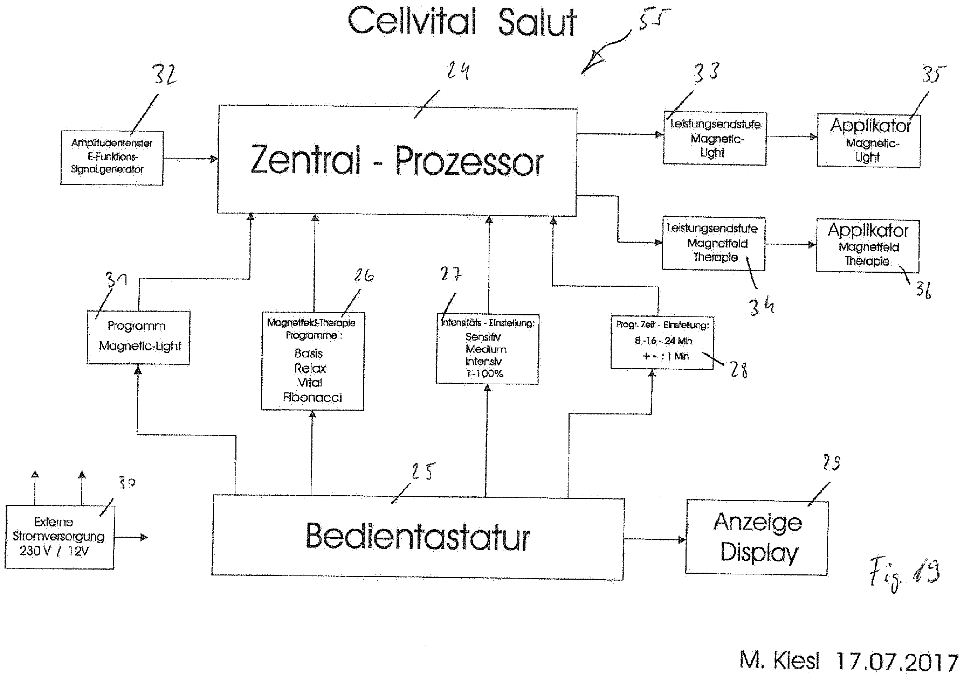

Demgemäß kann mit dem nachfolgend in Figur 19 gezeigten Behandlungsgerät die Intensität entsprechend der Figur 9 eingestellt werden, wobei im gezeigten Ausführungsbeispiel der Spulenstrom beispielsweise in Perioden 9 von 360 Mikrosekunden wirkt und zwischen den Perioden jeweils eine Pause eingeschaltet ist.

Die Figur 10 zeigt als weiteren Stand der Technik, dass aus den in Figur 9 erzeugten Grundimpulsen 10 nunmehr einzelne Grundimpulse hintereinander folgend mit einer Dauer von zum Beispiel 20 Millisekunden erzeugt werden, und insgesamt eine Behandlungszeit 11 von zum Beispiel 8 Sekunden gegeben ist. Nach Ablauf der Behandlungszeit 11 erfolgt dann eine Pausenzeit 12 von zum Beispiel 4 Sekunden, und danach beginnt wieder eine Behandlungszeit 11 von 8 Sekunden.

Die hier angegebenen Zeiten sind völlig beispielhaft zu verstehen und beschränken die Erfindung in keiner Weise. Es ist im Übrigen auch dargestellt, dass die Grundimpulse 10 mit einer Zeitdauer von 20 Millisekunden aufeinanderfolgen. Die Erfindung geht jedoch von dem Ausführungsbeispiel nach den Figuren 9 bis 11 aus und verbessert die dort dargestellten Funktionen in entscheidender Weise.

Aus der Figur 11 (Stand der Technik) ist erkennbar, dass aus den einzelnen Grundimpulsen 10 innerhalb der Behandlungszeit 11 nunmehr eine Vielzahl von Grundimpulsen 10 gebildet werden, die insgesamt einen Behandlungsblock 14, bilden, der innerhalb der Behandlungszeit 11 abläuft.

In einem solchen Behandlungsblock 14 ist dann beispielsweise eine Anzahl von 40 bis 60 Grundimpulsen 10 enthalten.

Hier setzt die Erfindung an, die in einem ersten Ausführungsbeispiel nunmehr gemäß Figur 12 vorsieht, dass die einzelnen Grundimpulse 10 nunmehr als rampenförmig ansteigende Grundimpulse 10a, 10b, 10c, 10d und 10e innerhalb einer Behandlungszeit 11 einer bestimmten Hüllkurve 17 folgen, die im gezeigten Ausführungsbeispiel als Parabelkurve ausgebildet ist. Die Amplituden dieser nacheinander folgenden Grundimpulse 10a-e folgen demnach einer Parabelkurve.

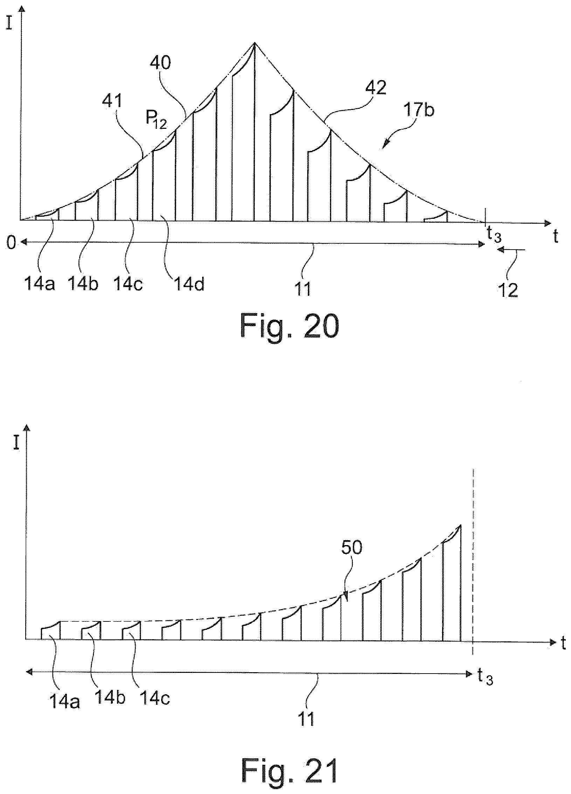

In einer anderen Ausgestaltung kann es vorgesehen sein, dass statt einer Parabelkurve der Amplituden einer Geraden folgen oder einer Exponentialkurve, wie anhand der Figuren 20 und 21 noch später dargestellt wird.

Wichtig ist, dass ausgehend von einem Startwert 16, der relativ gering ist und nahe bei Null liegt, nunmehr die Stromstärke und auch das Magnetfeld langsam entsprechend der verwendeten Hüllkurve 17 ansteigen, und zwar bis auf einen höheren Endwert 15.

1. Die Frequenz der Grundimpulse, die während einer Behandlungszeit (Haltezeit) erzeugt werden liegt im Bereich zwischen 1 Hz bis 50 Hz.

2. Der Startwert des Erstimpulses der angewendeten Grundimpulse beträgt liegt im Bereich zwischen 1% bis 30 % der maximalen Amplitude.

3. Die Amplitude des in der Behandlungszeit erzeugten Grundimpulses ist etwa 90-100% der maximalen Amplitude.

4. Die Behandlungszeit (Haltezeit) ist im Bereich zwischen 1s bis 12s und die dazwischen liegende Pausenzeit liegt im Bereich zwischen 4s bis 12s.

Die Figur 13 zeigt im Vergleich zu Figur 12 ein zweites bevorzugtes Ausführungsbeispiel, bei dem erkennbar ist, dass die Hüllkurve 17a sich nur über einen Teil der Behandlungszeit 11 als ansteigender Kurvenast 57 erstreckt, zum Beispiel nur bis zur Hälfte der Behandlungszeit 11, und dass ab der Überschreitung der hälftigen Behandlungszeit t1 nunmehr in Form des gleichbleibenden Kurvenastes 58 der maximale Spulenstrom bis zum Ende der Behandlungszeit 11 erzeugt wird.

Damit wird nur lediglich eine ansteigende Startrampe 18 in Form des ansteigenden Kurvenastes 57 verwendet, in deren Verlauf die Hüllkurve 17 verwendet wird, die - entsprechend der vorherigen Beschreibung - entweder als Parabelkurve, als Gerade oder als E-Funktion ausgebildet sein kann.

Vorteil dieser Maßnahme ist, dass ab der Zeit t1 bis zur Zeit t2 - im Bereich des gleichbleibenden Kurvenastes 58 - der volle Spulenstrom und damit das maximale Magnetfeld zur Verfügung stehen, während in der Zeit von 0 bis t1 eine ansteigende Startrampe 18 verwendet wird, um so ein langsames Einschleichen des Magnetfeldes in das Gewebe zu erreichen. Damit kommt es nicht mehr zu unangenehmen Gewebereaktionen und nicht mehr zu nervalen Störimpulsen, die den Behandlungserfolg stark beeinträchtigten.

Die Figur 14 zeigt nun, dass die Vielzahl der Grundimpulse 10 mit ihren jeweiligen Hüllkurven entsprechend den Kurvenästen 57, 58, einen Behandlungsblock 14 bilden und eine Vielzahl von Behandlungsblöcken 14 in Figur 14 ein Paket bildet, und diese Pakete in der Ausführungsform nach Figur 14 mit gleicher Amplitude nacheinander ablaufen. Alle in Figur 13 dargestellten Grundimpulse bilden somit Impulspakete mit gleichbleibender Amplitude, in denen jedoch die Grundimpulse 10 mit ansteigender Amplitude entweder nach Figur 12 oder Figur 13 enthalten sind.

Eine Vielzahl von Behandlungsblöcken 14, die jeweils aus einer Vielzahl von Grundimpulsen 10 bestehen sind demnach nach Figur 14 zu Impulspaketen 44 zusammengefasst. Der Zeitabstand 13 zwischen den einzelnen Grundimpulsen 10 ist damit zusammengeschrumpft und als Vielzahl von zeitlich hintereinander folgenden Impulspaketen 44 in Figur 14 und 15 dargestellt. Es kann mit dem in Figur 19 dargestellten Steuergerät 55 sowohl die Pausenzeit 12 eingestellt werden als auch die Behandlungszeit 11. Im gezeigten Anwendungsbeispiel erstreckt sich die Behandlungszeit 11 mit den Impulspaketen 44 über eine Dauer von 8 Sekunden und danach erfolgt jeweils eine Pause von z.B. 4 Sekunden.

In dem Ausführungsbeispiel nach Figur 14 ist die Hüllkurve der Stromamplituden als Rechteckkurve ausgebildet. Nachdem aber in jedem Impulspaket 44 eine Vielzahl von rampenförmig ansteigenden Grundimpulsen enthalten ist, kommt es zu einem guten Therapieerfolg.

Die verwendeten Grundimpulse 10, 10a, 10b weisen bevorzugt folgende Eigenschaften auf:

| | |
|---|---|
| Envelope Pulsform (Rechteck, e-Funktion oder Sägezahn) | |
| Repetitionsfrequenz | (1 Hz - 50Hz) |
| Startwert Erstimpuls | (1% - 100%) |
| Endwert Letztimpuls | (1% - 100%) |
| Haltezeit | (1s - 12s) |
| Pausezeit | (4s - 12s) |

Der Therapieerfolg kann jedoch noch gesteigert werden, wenn anstatt einer rechteckigen Hüllkurve gemäß Figur 15 nunmehr eine rampenförmig ansteigende Hüllkurve 20 verwendet wird.

Diese Hüllkurve 20 besteht aus einem ansteigenden Kurvenast 67, dessen Anstieg nahe Null beginnt und der sich über mehrere Behandlungs- und Pausenzeiten 11, 12 erstreckt. Der ansteigende Kurvenast 67 wird auch als "Therapierampe" bezeichnet.

In Figur 15 ist als Abwandlung zu der Figur 14 noch dargestellt, dass die einzelnen Amplituden der nacheinander folgend ablaufenden Behandlungspakete 14 einer Hüllkurve 17 folgen können, sodass auch die einzelnen nacheinander folgenden Behandlungsblöcke 14a-14e einer bestimmten Amplitudenfunktion folgen, nämlich einer Therapierampe 19, die von einer ansteigenden Hüllkurve 20 gebildet ist.

Auch diese Hüllkurve 20 kann entweder als Gerade ausgebildet sein oder als Parabel oder als E-Funktion.

Es wird ein langsames Einschleichen der einzelnen, aus Grundimpulsen bestehenden Behandlungsblöcke 14 erreicht, wobei in jedem Behandlungsblock 14 wiederum eine Vielzahl von Grundstromimpulsen 10 enthalten sind, von denen jeder der Hüllkurvenfunktion nach Figur 13 entspricht.

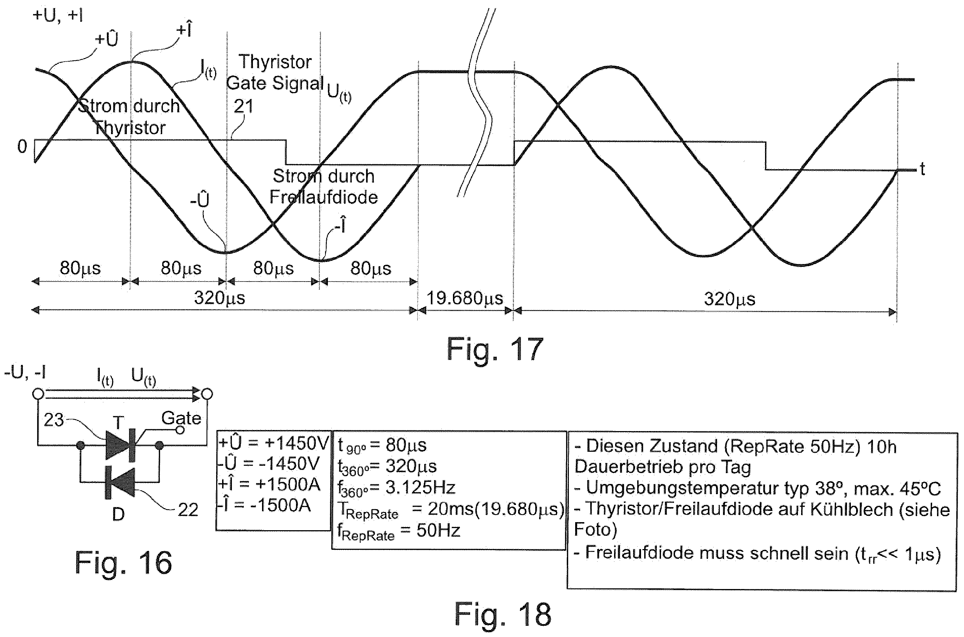

Die Figuren 16 und 17 zeigen die technische Ausführung für die Stromsteuerung nach den vorher genannten Figuren 13, 14 und 15.

Aus Figur 17 ergibt sich, dass ein Thyristor 23 an seinem Gate mit einem Gate-Signal 21 gemäß Figur 16 angesteuert wird, und eine entgegengesetzt polarisierte Freilaufdiode 22 parallel geschaltet ist.

Von dem Zeitpunkt 0 ausgehend, ergibt sich somit ein positiver Spitzenstrom von zum Beispiel 1500 Ampere, der bei einer bestimmten Zeit den Nulldurchgang erreicht, und dann erfolgt ein Ausgleichsstrom als Strom durch die Freilaufdiode als negativer Strom -1500 Ampere.

Ebenso sind die Spannungsverläufe an der Spule eingezeichnet und die bevorzugten Zeitabstände, die dort wirken.

In der Figur 18 (Tabelle) sind die verschiedenen Parameter im Einzelnen nach Zahlenwerten aufgeführt.

Es wird betont, dass die Zahlenwerte nur ein bevorzugtes Ausführungsbeispiel darstellen und die Erfindung dadurch nicht beschränkt ist.

In Figur 19 ist der Funktionsaufbau eines solchen Steuergerätes 55 dargestellt, wobei ein Signalgenerator 32 eine digitalen Zentralprozessor 24 ansteuert, mit dem das Amplitudenfenster und die Hüllkurven 17, 20 erzeugt werden.

Im Zentralprozessor 24 ist ein Eingang für einen Programmwähler 31 geschaltet, mit dem der Lichteffekt über den Applikator 35 erzeugt werden kann. Dieser wird wiederum von einer Bedientastatur 25 angesteuert.

Die Bedientastatur 25 steuert ebenfalls einen Programmwähler 26 an, mit dem die verschiedenen Programme der Magnetfeldtherapie angesteuert werden können.

Neben einem Basisprogramm gibt es ein Relax-Programm, ein Vitalprogramm und ein Fibonacci-Programm, bei dem eine bestimmte erfindungsgemäße Frequenzverteilung der Magnetimpulse angewendet wird.

Die Bedientastatur 25 steuert auch einen Intensitätswähler 27 an, mit dem die Intensität der Magnetpulse gesteuert werden kann. Es gibt dabei verschiedene Programme.

Die Bedientastatur 25 steuert auch eine Zeiteinstellung 28 an, und beim Ausgang des Zentralprozessors 24 wird eine Leistungsendstufe 33 für die Lichttherapie am Applikator 35 angesteuert, und ebenso eine Leistungsendstufe 34 für die Magnetfeldtherapie, die schließlich dem Applikator 36 zugeführt wird, der im gezeigten Ausführungsbeispiel als Behandlungsgerät 1 mit seinen Spulen 5, 5a, 5b ausgebildet sein kann.

Es ist noch eine Stromversorgung 30 vorhanden und eine Anzeige 29 für die Anzeige der verschiedenen Funktionszustände.

Die Figur 20 zeigt in Abwandlung zu der erfindungsgemäßen Rampenfunktion im Vergleich zu Figur 12, dass auch eine andere Rampenfunktion für die Behandlungsblöcke 14 erzielt werden kann.

Dort steigen die Amplituden der Behandlungsblöcke 14a, 14b, 14c, 14d in der Art eines exponentiell ansteigenden Kurvenzweiges 41 bis zu einer mittleren Behandlungszeit an und fallen in der Mitte der Behandlungszeit 11 wieder als weiterer Kurvenzweig 42 exponentiell ab, bis die Zeit t3 erreicht wird. Danach schließt sich die Pausenzeit 12 an.

Statt einer Startrampe 18 mit einer langsam ansteigenden Hüllkurve 17, 17a kann deshalb auch eine E-Funktion verwendet werden, die aus einem ansteigenden Kurvenzweig 41 und einem abfallenden Kurvenzweig 42 besteht.

In Abwandlung zum Ausführungsbeispiel nach Figur 20 zeigt die Figur 21, dass statt eines ansteigenden und eines abfallenden Kurvenzweiges 41, 42 auch nur ein ansteigender Kurvenzweig vorhanden sein kann, und die daraus gebildete Hüllkurve 50 einer Exponentialfunktion entspricht.

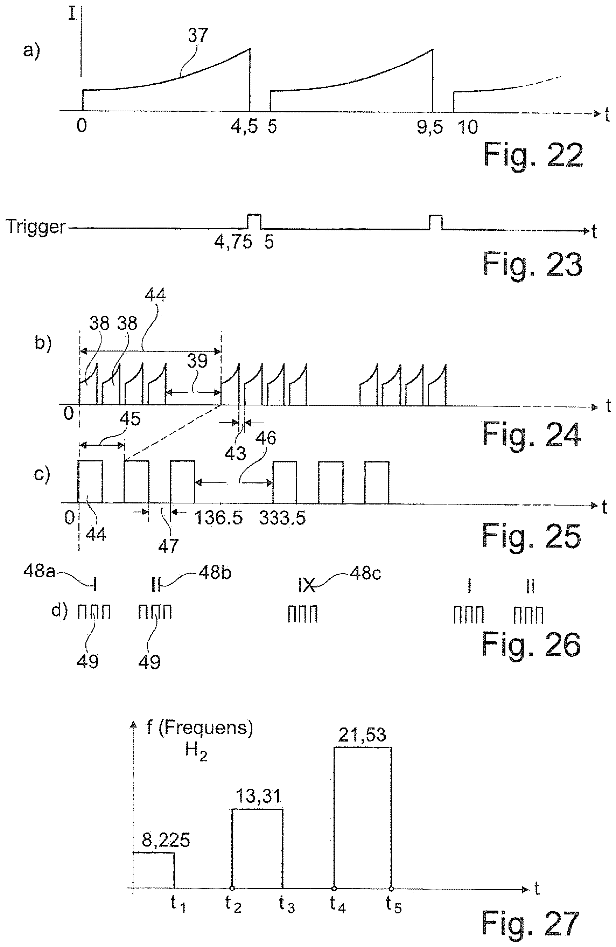

Die Figuren 22 bis 27 zeigen nun, dass alle Grundimpulse und die daraus gebildeten Behandlungsblöcke 14 einem bestimmten Frequenzmuster folgen, welches als Fibonacci-Frequenzverteilung bezeichnet wird.

Dabei gibt es eine Vielzahl von Ausführungsformen, die nur annähernd nachfolgend in einem Ausführungsbeispiel beschrieben werden.

Die Anwendung von Frequenzen aus der Fibonacci-Reihe lässt folgende Ausführungsformen zu, wobei jedes Ausführungsbeispiel mit jedem anderen Ausführungsbeispiel oder auch mit mehreren Ausführungsbeispielen untereinander kombiniert werden kann:

1 Anwendung einer oder mehrerer Fibonacci-Frequenzen für die Ausbildung der Grundimpulse 10, 37 während der gesamten Anwendungszeit oder für einen Teil der Anwendungszeit.

2 Anwendung einer oder mehrerer Fibonacci-Frequenzen für die Ausbildung der aus den Grundimpulsen 10, 37 gebildeten Impulspakete 44 während der gesamten Anwendungszeit oder für einen Teil der Anwendungszeit.

3 Anwendung einer oder mehrerer Fibonacci-Frequenzen für die Ausbildung der aus den Impulspaketen 44 gebildeten Impulspakete 45, 49 während der gesamten Anwendungszeit oder für einen Teil der Anwendungszeit.

4 Anwendung einer oder mehrerer Fibonacci-Frequenzen für die Ausbildung der Frequenz der aufeinander folgenden Pausenzeiten 39, 43, 46, 47 während der gesamten Anwendungszeit oder für einen Teil der Anwendungszeit.

5 Anwendung einer oder mehrerer Fibonacci-Frequenzen für die Ausbildung der Frequenz der aufeinander folgenden Behandlungszeiten 10, 11, 44, 45, 49 während der gesamten Anwendungszeit oder für einen Teil der Anwendungszeit.

Dazu zeigt die Figur 22 als Beispiel die rampenförmig ansteigenden Einzelimpulse 37, die dann nach Figur 23 von dazwischenliegenden Triggerimpulsen unterbrochen sind. Die Einzelimpulse 37 entsprechen den vorher gezeigten Grundimpulsen 10.

Die Dauer der Einzelimpulse 37 in Verbindung mit der Pausenzeit der ersten Pause zwischen 4,5-5 in Sekunden (Paketabstand 46) und den Pausenzeiten 9,5-10 Sekunden der 2. Pause bilden demzufolge eine bestimmte Frequenzreihe, die einer Fibonacci-Frequenz entspricht.

In Figur 24 ist dargestellt, dass aus den Einzelimpulsen 37 und Pausenzeiten aus Fig. 22 nunmehr gebildeten Impulspakete 38 in Verbindung mit den dazwischen liegenden Pausenzeiten mit einer bestimmten aufeinanderfolgenden Frequenz, nämlich einer Frequenz aus der Fibonacci-Reihe gebildet sind, und die Impulspakete 38 einen Pausen-Zeitabstand 39 aufweisen und sich mit einer bestimmten Frequenz aus der Fibonacci-Reihe periodisch wiederholen.

Aus den in Figur 24 dargestellten Impulspaketen 38 werden demzufolge - im Zeitmassstab dichtere - Impulspakete 44 gebildet, die auch weitere dazwischenliegende Pausenzeiten (Paketabstände 46, 47) beinhalten, und daraus bilden sich die noch größeren Impulspakete 44, die mit dazwischenliegenden Pausenzeiten die noch größeren Impulspakete 45 ausbilden.

Diese bilden bestimmte Frequenzmuster, wie mit den Zeitabständen in Figur 25 dargestellt ist. Der eingezeichnete Paketabstand 46 entspricht einer bestimmten Pausenzeit. Die Figur 26 zeigt die Frequenzverteilung der in Figur 25 dargestellten größeren Impulspakete 45 in einem noch dichteren Zeitmassstab.in Form von zeitlich verdichteten Impulspakten 49.

Daraus werden demzufolge die größeren Impulspakete 49 gebildet, die wiederum in einem bestimmten Frequenzabstand entsprechend mindestens einer Frequenz der Fibonacci-Reihe aufeinanderfolgen.

Die in Figur 22 dargestellten Pausenzeiten 4,5 - 5 und 9,5 -10 Sekunden der Einzelimpulse 10, 37 sind Pausen entsprechend einer Fibonacci-Verteilung.

Die in Figur 25 dargestellten Pausenzeiten 0-136,5; 333,5 bis XX sind wiederum Pausenzeiten einer Fibonacci-Verteilung.

Die Figur 27 zeigt nun, dass die Frequenzen der Grundimpulse 10, 37 bestimmte Werte einnehmen, nämlich im Zeitpunkt von 0 bis t1 eine Frequenz von 8,225 Hertz, im Zeitpunkt von t2 bis t3 eine Frequenz von 13,31 Hertz und im Zeitpunkt von t4 bis t5 eine Frequenz von 21,53 Hertz usw.

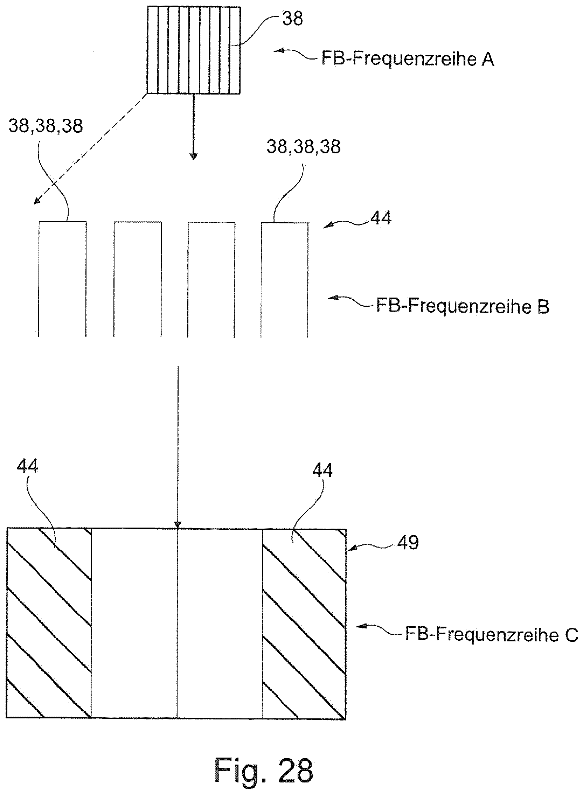

Die Figur 28 zeigt die Ineinanderschachtelung der verschiedenen Impulspakete 38 zu größeren Impulspaketen 44, und aus diesen wiederum noch größere Impulspakete 49, die in einem bestimmten Fibonacci-Frequenzmuster gebildet sind.

Die Aufeinanderfolge dieser Frequenzmuster ist in der Allgemeinen Beschreibung angegeben.

Die Figur 28 offenbart mehrere unterschiedliche Ausführungsformen bei der Anwendung der Frequenzverteilung nach Fibonacci (FB) der verwendeten Frequenzen:

Die Grundimpulse 10, 37 entsprechen einer ersten FB-Verteilung A

Die aus den Grundimpulsen 10, 37 gebildeten Impulspakete 44 entsprechen einer zweiten FB-Verteilung B

Die aus den Impulspaketen gebildeten Impulspakete 49 entsprechen einer dritten FB-Verteilung.

Für diese Ausführungsformen gelten dann die verschiedenen möglichen Frequenz-Verteilungen und deren Kombination untereinander nach FB: FB (A) und/oder FB (B) und/oder FB (C).

(1) Das bedeutet, dass nur die Frequenz der Grundimpulse 10, 37 einer FB-Verteilung entsprechen kann, dass aber die Frequenz der Impulspakte 44 und 49 gleichbleibend ist.

(2) Das bedeutet, dass die Frequenz der Grundimpulse 10, 37 einer FB-Verteilung entsprechen kann, dass die Frequenz der Impulspakte 44 ebenfalls einer FB-Verteilung entspricht, die Frequenz der Impulspakete 49 aber gleichbleibend ist.

(3) Das bedeutet, dass die Frequenz der Grundimpulse 10, 37 einer FB-Verteilung entsprechen kann, dass aber die Frequenz der Impulspakte 44 und 49 ebenfalls einer FB-Verteilung entspricht.

(4) Weitere Ausführungen ergeben sich durch die weiteren Mutationen der vorgenannten Auswahlmöglichkeiten A und/oder B und/oder C

Dabei kann die Frequenzverteilung (A) gleich oder ungleich der Frequenzverteilung (B) gleich oder ungleich der Frequenzverteilung (C) sein.

Somit wird klar, dass die bestimmte Häufigkeitsverteilung der Grundimpulse über den Zeitablauf der Fibonacci-Frequenzverteilung folgt, und damit der Behandlungserfolg mit Magnetfeldern 6 im Behandlungsgerät 1 wesentlich gesteigert werden kann.

Unter den oben genannten Begriff Fibonacci-Frequenzverteilung sind allgemein die in der Tabelle 1 angegebenen Frequenzen gemeint. Jede dieser Frequenzen kann in Alleinstellung für die Frequenzverteilung (A) und/oder (B) und/oder (C) verwendet werden. Das bedeutet, dass jede der in Tabelle 1 genannte Fibonacci-Frequenz als Frequenz für die Grundimpulse 10, 37 (Verteilung A)) und/oder für die Frequenz der aus den Grundimpulsen 10, 37 gebildeten Impulspakete 44 und/oder für die aus den Impulspaketen 44 gebildeten Impulspaketen 49 verwendet werden kann.

Selbstverständlich ist es möglich, für die Grundimpulse 10, 37 eine erste Fibonacci-Frequenz (A), für die Impulspakte 44 eine zweite Fibonacci-Frequenz (B) und für die Impulspakte 49 eine dritte Fibonacci-Frequenz (C) zu verwenden.

Der Begriff "Frequenzverteilung" bedeutet deshalb, dass jede beliebige Frequenz (A und/oder B und/oder C) aus der Fibonacci-Frequenzreihe in jeder beliebigen Verteilung (Größe) für die Grundimpulse 10, 37 und/oder die Impulspakte 44 und/oder die daraus gebildeten Impulspakete 49 verwendet werden kann.

Diese Frequenzen können auch der *Fibonacci*-**Frequenzskalierung:** 8,225 13,31 21,53 Hz entsprechen.

Es ist vorteilhaft, wenn alle hier verwendeten Impulse 10, 37; und die daraus gebildeten Impulspakete 44 und 49 einen rampenförmigen Anstieg aufweisen, wie dies in den Figuren 13 bis 15 dargestellt ist. Auf die dortige Beschreibung wird verwiesen.

Damit wird der Vorteil erreicht, dass die physiologisch für das Körpergewebe besonders verträglichen und besonders wirksamen Fibonacci-Impulse (das sind die Impulse 10, 37 und die Impulspakte 44 und 49 mit der jeweils ausgewählten Fibonacci-Frequenz A, B, C) einen verbesserten Therapieeffekt bei verkürzter Behandlungszeit erbringen.

In der Allgemeinen Beschreibung wurde bereits schon - in Verbindung mit Tabelle 1 - angegeben, dass die Verteilung der Intensität der Magnetfelder entsprechend der Fibonacci-Frequenzskalierung besondere Einflüsse auf die Herzfrequenz hat.

Damit kann mit der Anwendung der Fibonacci-Frequenzskalierung sowohl die Herzfrequenz, die Durchblutungsstärke, das Füllvolumen des Herzbeutels und andere physiologische Parameter des Blutkreislaufes positiv beeinflusst werden, ebenso wie die Beeinflussung von Strömen in den übrigen Körperorganen.

Es hat sich als besonders vorteilhaft herausgestellt, wenn die in der Rückenlehne 2 angeordnete Behandlungsspule 5a bis auf eine Frequenz von 1 Hertz heruntergeregelt werden kann, weil dann damit eine effektive strominduzierte Massage des Herzbeutels möglich ist, und damit das Füllvolumen des Herzbeutels und andere physiologische Parameter verbessert werden können.

Das Zentralinstitut für Medizintechnik der Technischen Universität München hat für den in Figur 8 dargestellten Behandlungsstuhl 1 magnetischen Flussdichten an verschiedenen Koordinatenpunkten gemessen.

Es handelt sich um einen Behandlungsstuhl 1, der beispielsweise zur Inkontinenz-Behandlung verwendet wird. Das Behandlungsprinzip besteht im Wesentlichen aus der repetitiven, magnetischen Stimulation der Beckenbodenmuskulatur. Die Methodik entspricht weitestgehend der, der etablierten transkraniellen magnetischen Stimulation.

Die messtechnische Aufgabe bestand in der Messung des Betrages der magnetischen Flussdichte $|B|$ in der Einheit Tesla an verschiedenen Koordinaten, sowie in der Messung des zeitabhängigen Spulenstroms der Erregerspule.

Es wurden die folgenden Messebenen festgelegt:

- 400 Messpunkte in der Sitzebene ($z = 0$) bei Intensitätseinstellung 60%
- 400 Messpunkte in der Ebene des Beckenbodens ($z = 12$ cm) bei Intensitätseinstellung 60%
- 400 Messpunkte in der Ebene des Herzens ($z = 35$ cm) bei Intensitätseinstellung 60%
- 1 Messpunkt in der Ebene des Gehirns ($x = 0$, $y = 0$, $z = 65$ cm) bei Intensitätseinstellung 60%
- Messung des Zeitverlaufs des Spulenstroms bei den Intensitätseinstellungen 20%, 40%, 60%, 80% und 100%
- Messung des Zeitverlaufs der magnetischen Flussdichte bei der Intensitätseinstellung 60% an 5 Messpunkten bei $y = 0$ und $z = 0$, $z = 12$ cm und $z = 65$ cm

Alle Messungen wurden bei einer Stimulationsfrequenz von 5 Hz durchgeführt.

Koord inatensystem

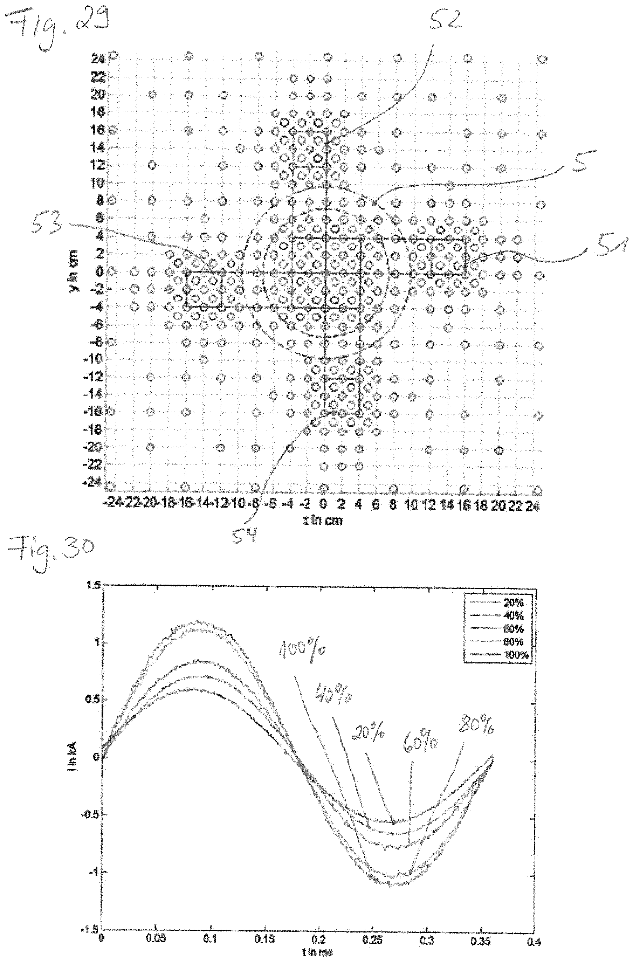

Figur 29 zeigt die Verteilung von 400 Messpunkten in einer x-y Ebene, beispielsweise der Ebene der Sitzfläche 3 des Behandlungsstuhls 1 von Figur 8. Die unterbrochenen Linien zeigen die Lage der Erregerspule, welche der Behandlungsspule 5 des Behandlungsstuhls 1 entspricht, sowie die horizontalen verlaufenden Schenkel der vier U-förmigen Magnetkerne 51, 52, 53, 54. Die durchgezogenen Linien markieren die Positionen der stirnseitigen Polflächen der Magnetkerne 51-54. Das Raster ist aufgrund der Geometrie der vorhandenen Magnetkerne in x- und y-Richtung unregelmäßig gewählt.

Die Behandlungsspule 5 weist eine ringartige Geometrie auf und ist an eine elektrische Stromversorgungseinrichtung, beispielsweise das oben beschriebene Steuergerät 55 angeschlossen, mittels dessen die Spule 5 mit einem geeigneten Stromsignal zur Erzeugung transienter Magnetfelder beaufschlagt werden kann.

Die Behandlungsspule 5 hat vorzugsweise eine torusartige Gestalt. Es sind vier in Umfangsrichtung der Behandlungsspule 5 jeweils um 90° versetzt bzw. 90° verdreht zueinander ausgerichtete Magnetkerne 51-54 mit U-förmiger Gestalt vorgesehen. Jeder dieser Magnetkerne 51-54 weist einen quadratischen bzw. rechteckigen Querschnitt auf. Sämtliche Magnetkerne 51-54 weisen an ihren nach oben ragenden Schenkeln eine im Wesentlichen planare Polfläche auf.

Die planaren Polflächen der einzelnen Kerne 51-54 liegen im gezeigten Ausführungsbeispiel allesamt in einer Ebene, die mit der Ebene der Behandlungsspule 5 zusammenfallen kann. Dadurch, dass jeweils einer der nach oben ragenden Endabschnitte der Magnetkerne 51-54 innerhalb der Behandlungsspule 5 liegen, bildet jeder Magnetkern 51-54 an seinen Endabschnitten nach Stromrichtung innerhalb der Behandlungsspule 5 einen magnetischen Nordpol bzw. Südpol aus, während die außerhalb der Behandlungsspule 5 liegenden Endabschnitte einen magnetischen Südpol bzw. Nordpol ausbilden. Dementsprechend verlaufen zwischen den planaren Endabschnitten kreisbogenartige magnetische Feldlinien.

Die Anordnung, bestehend aus der Behandlungsspule 5 und den Magnetkernen 51-54, ist beispielsweise unterhalb der Sitzfläche 3 des Behandlungsstuhls 1 angeordnet. Dadurch können die von den freien Endabschnitten der Magnetkerne 51-54 ausgehenden magnetischen Felder vergleichsweise tief in das zu behandelnde biologische Gewebe eines Patienten eindringen.

Messmittel

Für die Messung der magnetischen Flussdichten wurden die nachfolgenden Messinstrumente verwendet:

- Strommesszange i3000s, Fluke, Deutschland
- Oszilloskop Wave Surfer 44 MXs, Le Croy, USA
- DSP Gaussmeter 455, LakeShore, USA
- Axial Sonde HMNA-1904-VR, LakeShore, USA
- Transversal Sonde HMNT-4E04-VR, LakeShore, USA
- Suchspule, IND-001, TUM, Deutschland

Die Messmittel wurden entsprechend den Herstellerangaben kalibriert. Insbesondere die Sonden für die Messung des Magnetfeldes wurden zusätzlich temperaturkalibriert.

Durchführung der Messung

An dem Behandlungsstuhl 1 wurden zunächst die Lederpolster entfernt und die Abdeckung der Stimulationseinheit demontiert. Im Anschluss wurde mittels einer Strommesszange der Strom in der Behandlungsspule 5 bei den Intensitätseinstellungen 20%, 40%, 60%, 80% und 100% bei einer Repetierfrequenz von 5 Hz gemessen.

Anschließend wurden die Polster wieder montiert und die magnetische Flussdichte in den drei Raumrichtungen an jedem Koordinatenpunkt (Figur 29) jeder Ebene (z = 0 cm, z = 12 cm, z = 35 cm) gemessen. Der Betrag der magnetischen Flussdichte errechnet sich aus den Beiträgen der einzelnen Richtungskomponenten zu:

$$|B| = \sqrt{B_x^2 + B_y^2 + B_z^2} \qquad (13)$$

Zur Bestimmung der Amplituden der magnetischen Flussdichte B an jedem Koordinatenpunkt wurde jeweils über 100 Stimulationspulse das Maximum der magnetischen Flussdichte ermittelt.

Der Fehler, der bei der Positionierung der Messsonden und durch das Messverfahren auftrat, wurde mit allen Sonden über eine Zeit von 20 s (dies entspricht 100 Stimulationspulse bei einer Repetierfrequenz von 5 Hz) bestimmt. Der Fehler beträgt im Durchschnitt etwa 2% vom Maximalwert. Die Fehlerverteilung wurde auf Normalverteilung mit Hilfe des Shapiro-Wilk-Tests getestet (S. S. Shapiro, M. B. Wilk, "An Analysis of Variance Test for Normality (Complete Samples)", Biometrica, Vol. 52, No. 3-4, pp. 591-611). Aufgrund der Normalverteilung dieses Fehlers wurden die Messergebnisse diesbezüglich fehlerbereinigt.

Messung des Spulenstroms

Figur 30 zeigt den gemessenen Spulenstrom der Behandlungsspule 5 bei den Intensitätseinstellungen 20%, 40%, 60%, 80% und 100%. Der Spulenstrom hat einen gedämpften sinusoiden Zeitverlauf.

Der Zeitverlauf des Stromes folgt der Funktion

$$I(t) \propto sin(2\pi f \cdot t)e^{-\delta t} \qquad (14)$$

Zur Bestimmung der Länge des Stimulationspulses, der Schwingfrequenz und der Systemdämpfung wurden die Messwerte an die Formel 14 angefittet. Hierzu wurde die Methode der kleinsten Fehlerquadrate angewendet. Demnach beträgt die Länge eines Stimulationspulses ca. 360 $\mu$s. Diese Pulslänge entspricht einer Schwingfrequenz f = 2777 Hz. Die Systemdämpfung $\delta$ beträgt 499. Die Systemdämpfung resultiert überwiegend aus den ohmschen Verlusten in den Spulenleitungen. Diese Parameter hängen nicht von der Intensitätseinstellung ab.

Dreidimensionale Verteilung der Flussdichte

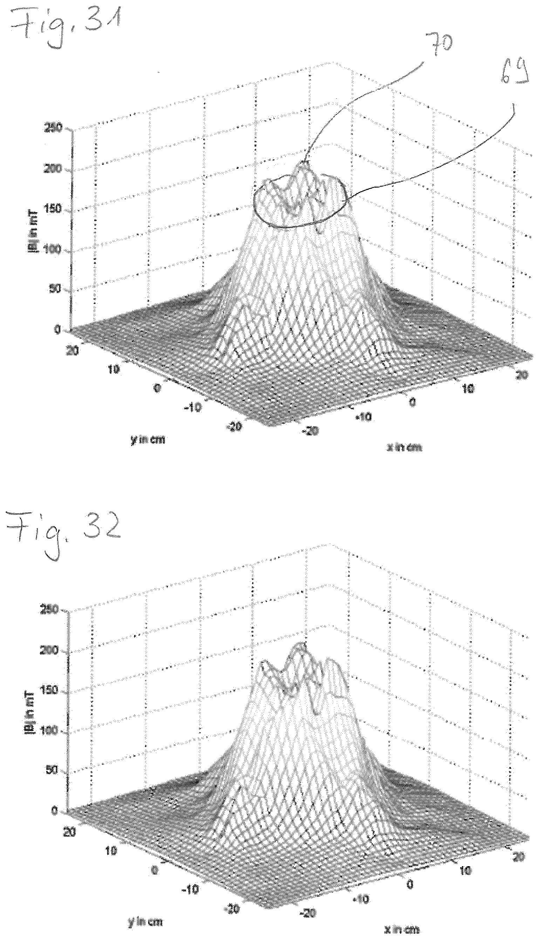

Die Figuren 31 bis 33 zeigen die räumliche Verteilung des Betrages der magnetischen Flussdichte in Millitesla bei der Intensitätseinstellung 60% in verschiedenen Ebenen.

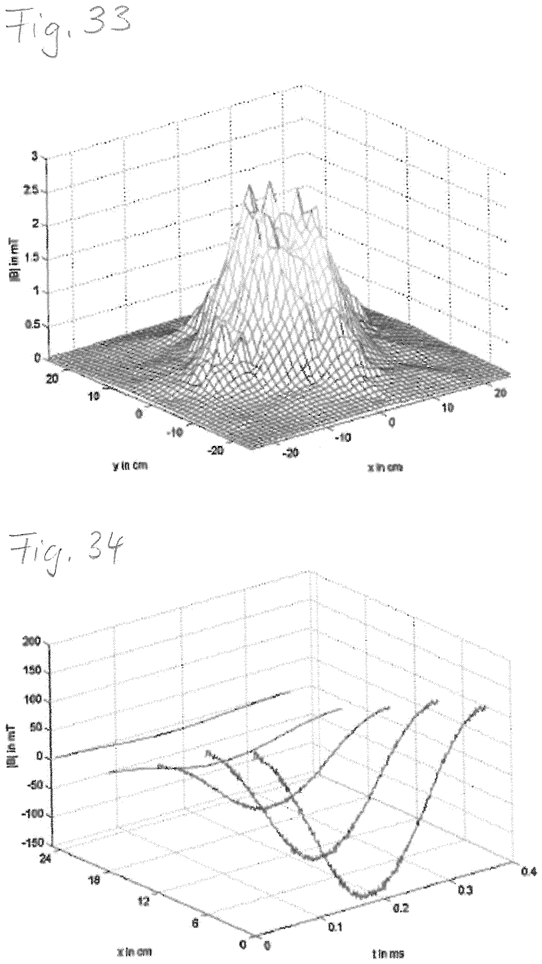

Figur 31 zeigt die räumliche Verteilung der magnetischen Flussdichte in der Sitzebene (z = 0). Figur 32 zeigt die räumliche Verteilung der magnetischen Flussdichte in der Beckenbodenebene (z = 12 cm). Figur 33 zeigt die räumliche Verteilung der magnetischen Flussdichte in der Herzebene (z = 35 cm). Man erkennt, dass die magnetische Flussdichte relativ steil ansteigt und im Maximum eine große wirksame Fläche mit relativ konstanter Flussdichte bildet.

Durch die vier U-förmigen Magnetkerne 51-54 der Behandlungsspule 5 hat das Magnetfeld ein ausgeprägtes räumliches Volumen, d.h. es ist pyramidenförmig und füllt eine Plateaufläche (69) in x- und y-Richtung mit relativ homogener Flussdichte aus. Dadurch kann das Magnetfeld mit gleichmäßiger Flussdichte tief und auf einer großen Fläche in das Gewebe des Patienten eindringen. In dieser Plateaufläche 69 beträgt die magnetische Flussdichte mindestens 85% des momentanen Spitzenwertes 70 der magnetischen Flussdichte.

Die Verteilung der Flussdichte über die Fläche ist in den betrachteten Messebenen gemäß den Figuren 31 bis 33 nahezu identisch, wobei lediglich die maximale Intensität mit zunehmendem Abstand z von der Sitzebene 3 abnimmt.

Flussdichte in Kopfhöhe

Die magnetische Flussdichte in Kopfhöhe wurde direkt im Zentrum bei (x/y/z) = (0/0/65 cm) gemessen. Die maximale Flussdichte (Betrag) wurde bei einer Stimulationsintensität von 60% zu 0,11 mT ermittelt.

Dynamische Messung der magnetischen Flussdichte

Die Figuren 34 bis 36 zeigen die Zeitverläufe der magnetischen Flussdichten (Betrag) entlang der positiven x-Achse an fünf exemplarischen Koordinatenpunkten bei y = 0 und einer Intensitätseinstellung von 60%. Die Verläufe folgen ebenfalls dem Zusammenhang nach Formel 14. Allerdings wurde das Aufnahmezeitfenster um 90° gegenüber der Darstellung in Figur 31 versetzt. Die Dämpfung und die Schwingfrequenz entsprechen denen des Stroms.

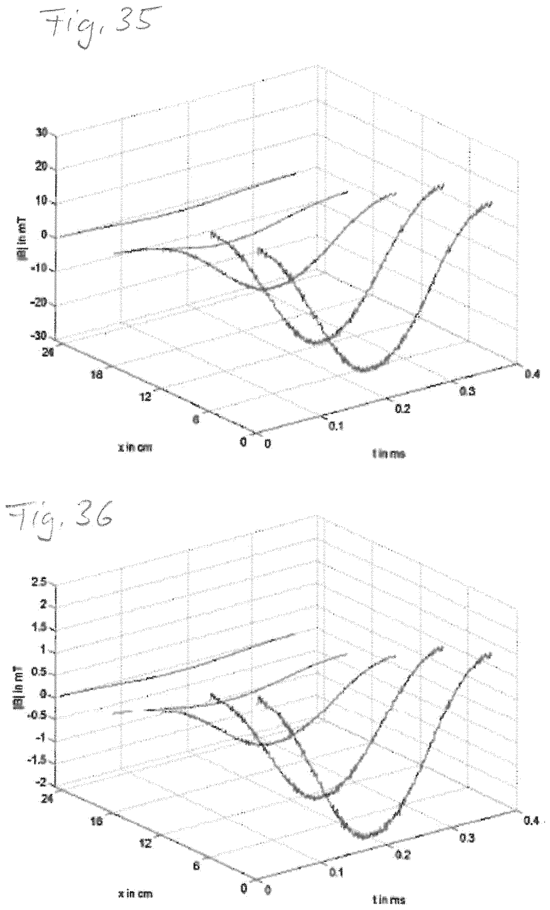

Figur 34 ist eine Darstellung der magnetischen Flussdichte (Zeitverlauf) in der Sitzebene (z = 0) entlang der positiven x-Achse an fünf Messstellen. Figur 35 ist eine Darstellung der magnetischen Flussdichte (Zeitverlauf) in der Beckenbodenebene (z = 12 cm) entlang der positiven x-Achse an fünf Messstellen. Figur 36 ist eine Darstellung der magnetischen Flussdichte (Zeitverlauf) in der Herzebene (z = 35 cm) entlang der positiven x-Achse an fünf Messstellen.

Zusammenhang zwischen Intensität und magnetischer Flussdichte

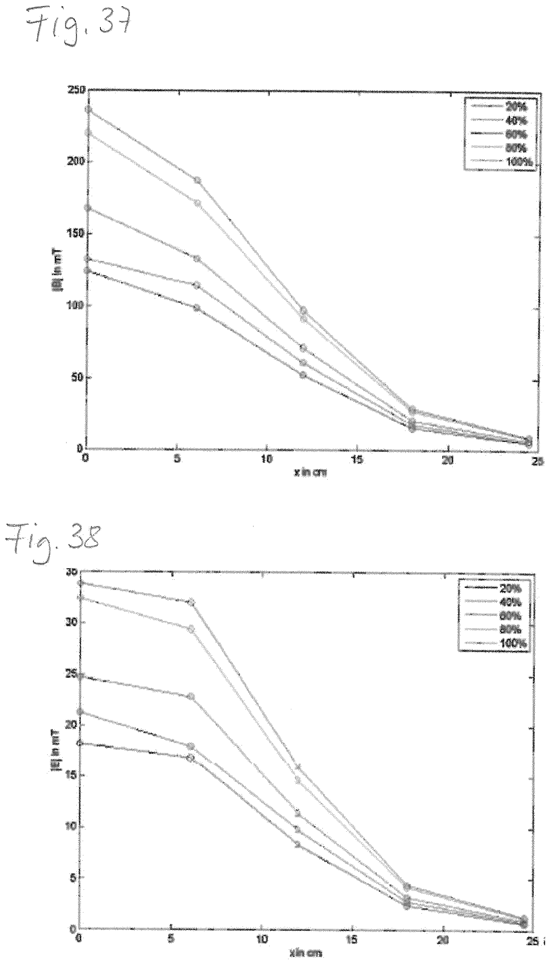

Die Figuren 37 bis 39 zeigen die gemessenen Werte für den Betrag der magnetischen Flussdichte an fünf exemplarischen Messpunkten entlang der positiven x-Achse bei y = 0 für die Sitz-, Beckenboden- und Herzebene bei verschiedenen Intensitätseinstellungen (20%, 40%, 60%, 80% und 100%).

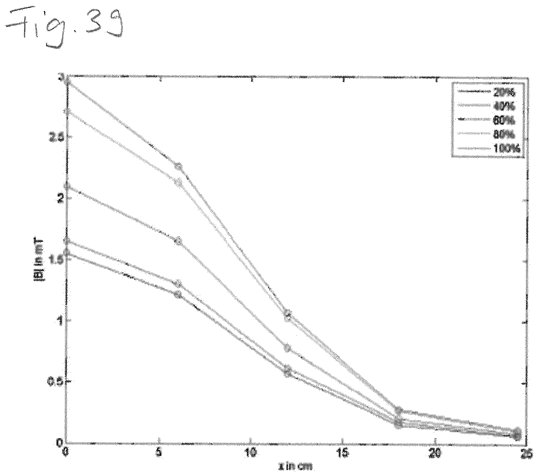

Figur 37 ist eine Darstellung der magnetischen Flussdichte in der Sitzebene (z = 0) entlang der positiven x-Achse an fünf Messstellen bei verschiedenen Intensitätseinstellungen. Figur 38 ist eine Darstellung der magnetischen Flussdichte in der Beckenbodenebene (z = 12 cm) entlang der positiven x-Achse an fünf Messstellen bei verschiedenen Intensitätseinstellungen. Figur 39 ist eine Darstellung der magnetischen Flussdichte in der Herzebene (z = 35 cm) entlang der positiven x-Achse an fünf Messstellen bei verschiedenen Intensitätseinstellungen.

Zusammenhang zwischen Spulenstrom und Repetierrate

Tabelle 2 zeigt die gemessenen maximalen Spulenströme bei den Intensitäten 20%, 40%, 60%, 80% und 100% bei den Repetierfrequenzen 5 Hz, 10 Hz und 30 Hz.

Tabelle 3 stellt die Abweichungen des Stromes bei 10 Hz und bei 30 Hz gegenüber der Frequenz 5 Hz dar (nur Abweichung der Mittelwerte). Damit ist eine Umrechnung der magnetischen Flussdichten auf andere Intensitäten und Frequenzen möglich.

| Intensität in % | 5 Hz (90 Pulse) | 10 Hz (120 Pulse) | 30 Hz (360 Pulse) |
|---|---|---|---|
| 20 % | 600 A ± 3,2 A | 610 A ± 18 A | 610 A ± 17,7 A |
| 40 % | 730 A ± 3,3 A | 740 A ± 20,2 A | 700 A ± 19,7 A |
| 60 % | 880 A ± 8,9 A | 830 A ± 17,9 A | 800 A ± 19,8 A |
| 80 % | 1180 A ± 20,7 A | 1180 A ± 31,6 A | 1060 A ± 20,7 A |
| 100 % | 1250 A ± 16,9 A | 1220 A ± 17,4 A | 1220 A ± 29,4 A |

Tabelle 2: Maximaler Spulenstrom bei verschiedenen Intensitäten und bei verschiedenen Frequenzen.

| Intensität in % | 10 Hz | 30 Hz |
|---|---|---|
| 20 % | + 2 % | + 2 % |
| 40 % | + 1 % | - 4 % |
| 60 % | - 6 % | - 9 % |
| 80 % | ± 0 % | - 10 % |
| 100 % | - 2 % | - 2 % |

Tabelle 3: Abweichung des maximalen Stroms bei 10 Hz und 30 Hz von dem Strom bei 5 Hz.

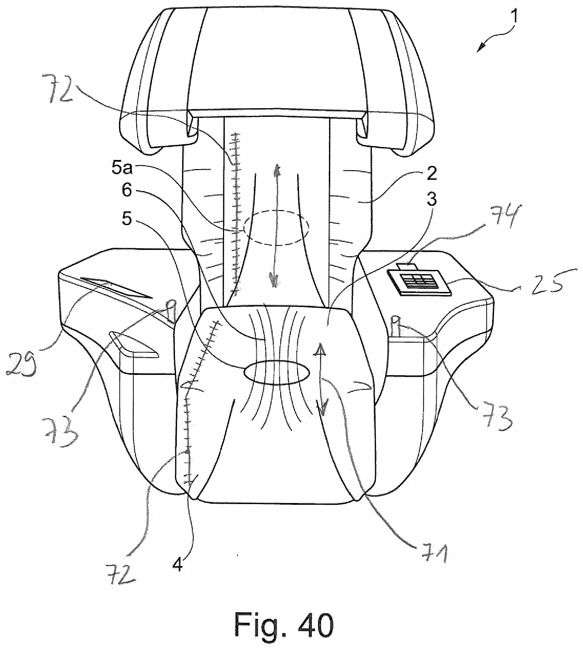

Figur 40 zeigt eine schematisierte Darstellung einer abgewandelten Ausgestaltung eines bevorzugten Behandlungsgerätes 1. Die Funktionen des Behandlungsgerätes 1 können auf den Patienten personalisiert werden, wobei die entsprechenden Behandlungsdaten zur Steuerung der Behandlungsspulen 5, 5a für die Magnetfeldtherapie, des Applikators 35 für die Lichttherapie (vgl. Figur 8) und gegebenenfalls eines zusätzlichen Sauerstoffgenerators zur Sauerstofftherapie auf einer persönlichen Chipkarte 74 oder ähnlichem gespeichert werden. Die Lichttherapie wird insbesondere zur Behandlung von Depressionen und die Sauerstofftherapie zur zusätzlichen Förderung des Muskelaufbaus eingesetzt. Die auf der Chipkarte 74 gespeicherten Behandlungsdaten, Einstellparameter und Messwerte kann sich der Benutzer bzw. der Therapeut auf der Anzeige 29 des Geräts jederzeit anzeigen lassen.

Im Gegensatz zur Figur 8 ist im Bereich der Sitzfläche 3 und des Fußteils 4 lediglich eine Behandlungsspule 5 angeordnet, die jedoch entlang der Sitzfläche 3 und des Fußteils 4 in Pfeilrichtung 71 verschiebbar angeordnet ist. Somit kann die Behandlungsspule 5 gezielt an die zu behandelnde Körperregion verfahren werden, z.B. in den Bereich des Beckenbodens, der Oberschenkel oder der Unterschenkel.

Um die einmal eingestellte und optimale Position der Behandlungsspule 5 immer wieder reproduzieren zu können, ist es vorgesehen, dass entlang der Sitzfläche 3 und des Fußteils 4 eine Skala 72 angeordnet ist, die entsprechende Ziffernangaben umfasst. Die eingestellte Position der Behandlungsspule 5 wird auf der Skala 72 angezeigt, beispielsweise mittels einer Leuchtanzeige. Alternativ oder zusätzlich kann die eigestellte Position der Behandlungsspule 5 auf der Anzeige 29 des Behandlungsgeräts angezeigt werden, beispielsweise durch einen numerischen Wert oder eine grafische Anzeige.

Entsprechend kann die Behandlungsspule 5a in der Rückenlehne 2 ebenfalls verschiebbar in Pfeilrichtung angeordnet sein, wobei die eingestellte Position entweder auf einer Skala 72 entlang der Rückenlehne 2 und/oder auf der Anzeige 29 angezeigt werden.

Die gewünschte(n) Position(en) der Behandlungsspulen 5 und 5a werden vorzugsweise auf der persönlichen Chipkarte 74 des Patienten gespeichert. Wenn der Patient sich mit seiner Chipkarte 74 am Behandlungsgerät 1 identifiziert, werden die persönlichen Einstellungen in das Behandlungsgerät 1 eingelesen und die Behandlungsspulen 5, 5a fahren automatisch in die auf der Chipkarte 74 gespeicherten individuellen Positionen. Diese Positionen können natürlich manuell jederzeit geändert und neu auf der Chipkarte 74 abgespeichert werden.

Bei der in der Rückenlehne 2 befindlichen Behandlungsspule 5a wird auch automatisch die Magnetfeldstärke reduziert, sobald die Behandlungsspule 5a in den Bereich des Herzens des Patienten kommt, das wird bei der ersten Behandlung vom Therapeuten eingestellt und auf der Chipkarte 74 programmiert. Die Chipkarte kann deshalb auch nicht übertragen werden. Der Behandlungszyklus umfasst gewöhnlich zwischen 10 und 20 Behandlungen.

An beiden Seitenteilen des Behandlungsgeräts 1 können Handgriffe 73 angeordnet sein, die in Figur 40 lediglich schematisch dargestellt sind. Die Handgriffe 73 sind vorzugsweise ergonomisch geformt und so angeordnet, dass die auf dem Behandlungsgerät 1 sitzende Person mit den Armen an den Handgriffen 73 ziehen kann. Die mit den Armen auf die Handgriffe 73 ausgeübte Kraft wird durch Meßaufnehmer in den Handgriffen 73 gemessen und auf der Anzeige 29 des Behandlungsgeräts 1 angezeigt und beispielsweise auf der persönlichen Chipkarte 74 zur nachfolgenden Auswertung aufgezeichnet. Dadurch kann der Kraftzuwachs beim Patienten durch die Magnetfeldbehandlung erfasst und dokumentiert werden.

**Zeichnungslegende**

| | |
|---|---|
| 1 | Behandlungsgerät |
| 2 | Rückenlehne |
| 3 | Sitzfläche |
| 4 | Fußteil |
| 5 | Behandlungsspule |
| 5a | Behandlungsspule |
| 5b | Behandlungsspule |
| 6 | Magnetfeld |
| 7 | Spulenstrom (Stufe 2) |
| 8 | Spulenstrom (Stufe 1) |
| 9 | Periode |
| 10 | Grundimpuls |
| 10a | Grundimpuls |
| 10b | Grundimpuls |
| 11 | Behandlungszeit |
| 12 | Pausenzeit |
| 13 | Zeitabstand (von 10) |
| 14 | Behandlungsblock |
| 15 | Endwert |
| 16 | Startwert |
| 17 | Hüllkurve (Parabel) |
| 17a | Hüllkurve |
| 17b | Hüllkurve |
| 18 | Startrampe |
| 19 | Therapierampe |
| 20 | Hüllkurve (groß) |
| 21 | Gate-Signal |
| 22 | Freilaufdiode |
| 23 | Thyristor |
| 24 | Zentralprozessor |
| 25 | Bedientastatur |
| 26 | Programmwähler |
| 27 | Intensitätswähler |
| 28 | Zeiteinstellung |
| 29 | Anzeige |
| 30 | Stromversorgung |
| 31 | Programmwähler |
| 32 | Signalgenerator |

| 33 | Leistungsendstufe |
|----|----|
| 34 | Leistungsendstufe |
| 35 | Applikator |
| 36 | Applikator |
| 37 | Einzelimpuls |
| 38 | Impulspaket (klein) |
| 39 | Abstand |
| 40 | Hüllkurve (groß) |
| 41 | Kurvenzweig ansteigend |
| 42 | Kurvenzweig abfallend |
| 43 | Impulsabstand |
| 44 | Impulspaket (groß) |
| 45 | Impulspaket (groß) |
| 46 | Paketabstand (groß) |
| 47 | Paketabstand (klein) |
| 48 | Frequenzfolge |
| 49 | Impulspaket |
| 50 | Hüllkurve (groß) |
| 51 | Magnetkern |
| 52 | Magnetkern |
| 53 | Magnetkern |
| 54 | Magnetkern |
| 55 | Steuergerät |
| 57 | Kurvenast (ansteigend) Fig. 13 |
| 58 | Kurvenast (gleichbleibend) Fig. 13 |
| 67 | Kurvenast (ansteigend) Fig. 15 |
| 68 | Kurvenast (gleichbleibend) Fig.15 |
| 69 | Plateaufläche |
| 70 | Spitzenwert (Flussdichte) |
| 71 | Pfeilrichtung |
| 72 | Skala |
| 73 | Handgriffe |
| 74 | Chipkarte |

**Patentansprüche**

1. Behandlungsgerät mit einem Magnetfeldapplikator (1, 55), der geeignet ist, einen rampenförmigen Signalverlauf der verwendeten Spulenströme (10, 37, 44, 49) zu erzeugen, der Signalverlauf bestehend aus niederfrequenten, in der Amplitude rampenförmig ansteigenden Grundimpulsen (10, 37) des Spulenstroms, die während einer definierten Behandlungszeit (11) wirken, die Bestandteil von aus den Grundimpulsen (10, 37) zusammengesetzten Impulspaketen (44) sind, deren durch die Amplituden der Grundimpulse (10, 37) beschriebene Hüllkurve (17, 17a, 17b) ebenfalls rampenförmig ausgebildet ist, wobei die Hüllkurve (17,a, 17b, 17c) der Amplituden der Grundimpulse (10, 37) einen von der Nähe von Null ausgehenden ansteigenden Kurvenast (57) ausbildet, der bis zur Mitte einer Behandlungszeit (11) ansteigt, **dadurch gekennzeichnet, dass** die Hüllkurve (17,a, 17b, 17c) der Amplituden der Grundimpulse (10, 37) von der Mitte bis zum Ende der Behandlungszeit (11) einen der maximalen Stromstärke entsprechend, gleichbleibenden Kurvenast (58) ausbildet, und dass das Behandlungsgerät Einrichtungen zur Lichttherapie (35) und / oder Sauerstofftherapie und / oder zur Kraftmessung (73) des Patienten aufweist, wobei die Kraftmesswerte gespeichert und auf der Anzeige (29) des Behandlungsgeräts (1) angezeigt werden.

2. Behandlungsgerät nach Anspruch 1, **dadurch gekennzeichnet, dass** eine Vielzahl von rampenförmig ansteigenden Grundimpulsen (10, 37) jeweils Impulspakete (44) bildet, deren Stromamplituden einer ansteigenden Hüllkurve (20) folgen.

3. Behandlungsgerät nach Anspruch 2, **dadurch gekennzeichnet, dass** die ansteigende Hüllkurve (20) aus einem ersten ansteigenden Kurvenast (67) besteht, der sich über mehrere Behandlungs- und Pausenzeiten (11, 12) erstreckt und der sich in einen gleichbleibenden, der maximalen Stromstärke entsprechenden weiteren Kurvenast (68) fortsetzt, der sich ebenfalls über mehrere Behandlungs-und Pausenzeiten (11, 12) erstreckt.

4. Behandlungsgerät nach Anspruch 1, **dadurch gekennzeichnet, dass** eine Vielzahl von rampenförmig ansteigenden Grundimpulsen (10, 37) jeweils Impulspakete (44) bildet, deren Stromamplitude einer Rechteckfunktion folgt.

5. Behandlungsgerät nach Anspruch 1, **dadurch gekennzeichnet, dass** die Frequenz der Grundimpulse (10, 37) in Hertz mindestens eine Frequenz der Fibonacci-Reihe 1, 2, 3, 5, 8, 13, 21, 34, 55, usw. ist.

6. Magnetfeldapplikator nach einem der Ansprüche 1 bis 5, **dadurch gekennzeichnet, dass** die Frequenz in Hertz der aus den Grundimpulsen (10, 37) gebildeten, aus Behandlungszeiten (11) und Pausenzeiten (12) bestehenden Impulspaketen (44) mindestens einer Frequenz der Fibonacci-Reihe 1, 2, 3, 5, 8, 13, 21, 34, 55, usw. entspricht.

7. Behandlungsgerät nach einem der Ansprüche 5 oder 6, **dadurch gekennzeichnet, dass** die Frequenz in Hertz der aus den Impulspaketen (44) gebildeten, aus Behandlungszeiten (11) und Pausenzeiten (12) bestehenden größeren Impulspaketen (45, 49) mindestens einer Frequenz der Fibonacci-Reihe 1, 2, 3, 5, 8, 13, 21, 34, 55, usw. entspricht.

8. Behandlungsgerät nach einem der Ansprüche 1 bis 7, **dadurch gekennzeichnet, dass** die Frequenz in Hertz der aufeinanderfolgenden Grundimpulse (10, 37) und/oder die Frequenz der aufeinanderfolgenden Impulspakete (44) und/oder die Frequenz der aus den Impulspaketen (44) gebildeten größeren Impulspaketen (45, 49) über die gesamte Anwendungszeit des Magnetfeldapplikators (1, 2, 5) variabel ist und mindestens einer oder mehreren der Frequenzen der Fibonacci-Reihe 1, 2, 3 , 5, 8, 13, 21, 34, 55, usw. entsprechen.

9. Behandlungsgerät nach einem der Ansprüche 1 bis 8, **dadurch gekennzeichnet, dass** die Frequenz in Hertz der aufeinanderfolgenden Behandlungszeiten (11) und oder der Pausenzeiten (12) über die gesamte Anwendungszeit des Magnetfeldapplikators (1, 2, 5) variabel ist und mindestens einer oder mehreren der Frequenzen der Fibonacci-Reihe 1, 2, 3, 5, 8, 13, 21, 34, 55, usw. entsprechen.

10. Behandlungsgerät nach einem der Ansprüche 1 bis 9, **dadurch gekennzeichnet, dass** der Spulenstrom in Perioden (9) von bis zu 360 Mikrosekunden wirkt, und zwischen den Perioden (9) jeweils eine Pause eingeschaltet ist.

11. Behandlungsgerät nach einem der Ansprüche 1 bis 10, **dadurch gekennzeichnet, dass** der Magnetfeldapplikator mindestens eine Behandlungsspule (5, 5a, 5b) mit einem Magnetkern (51-54) aufweist, die in ein Behandlungsgerät (1) eingebaut sind und ein aus einer Ebene (2, 3, 4) des Behandlungsgeräts (1) austretendes magnetisches Feld erzeugen.

12. Behandlungsgerät nach Anspruch 11, **dadurch gekennzeichnet, dass** das aus der Ebene (2, 3, 4) des Behandlungsgeräts (1) austretende Magnetfeld ein räumliches Volumen aufweist und eine Plateaufläche (69) in x- und y-Richtung der Ebene (2, 3, 4) mit homogener magnetischer Flussdichte ausfüllt.

13. Behandlungsgerät nach Anspruch 1, **dadurch gekennzeichnet, dass** der mindestens eine Magnetfeldapplikator (5, 5a, 5b) in einer Rückenlehne (2), Sitzfläche (3) oder einem Fußteil verschiebbar angeordnet ist, wobei dessen Position auf einer Skala (72) oder auf einer Anzeige (29) des Behandlungsgeräts (1) angezeigt wird.

14. Behandlungsgerät nach einem der Ansprüche 1 bis 13, **dadurch gekennzeichnet, dass** zur Aktivierung und Steuerung des Behandlungsgeräts (1) eine personalisierte Chipkarte (74) vorgesehen ist, auf welcher individuelle Daten, Einstellungen und Parameter des Patienten zur Inbetriebnahme und individuellen Steuerung des Behandlungsgeräts (1) gespeichert sind.

**Claims**

1. Treatment device with a magnetic field applicator (1, 55) which is suitable for generating a ramped signal pattern of the coil currents used (10, 37, 44, 49); the signal pattern consisting of low-frequency base pulses (10, 37) of the coil current rising like a ramp in amplitude which act during a defined treatment time (11), which are a component of pulse packets (44) composed of the base pulses (10, 37), the envelope (17, 17a, 17b) of which described by the amplitudes of the base pulses (10, 37) is likewise formed like a ramp, wherein the envelope (17a, 17b, 17c) of the amplitudes of the base pulses (10, 37) forms a curve branch (57) rising starting from close to zero which rises until the midpoint of a treatment time (11), **characterised in that** the envelope (17a, 17b, 17c) of the amplitudes of the base pulses (10, 37) from the midpoint to the end of the treatment time (11) forms a constant curve branch (58) corresponding to the maximum current strength, and **in that** the treatment device has arrangements for light therapy (35) and/or oxygen therapy and/or for strength measurement (73) of the patient, wherein the measured values of strength are stored and displayed on the display (29) of the treatment device (1).

2. Treatment device according to claim 1, **characterised in that** a plurality of base pulses (10, 37) rising like a ramp form respectively pulse packets (44), the current amplitudes of which follow a rising envelope (20).

3. Treatment device according to claim 2, **characterised in that** the rising envelope (20) consists of a first rising curve branch (67) which extends over several treatment times and break times (11, 12) and which continues in a constant further curve branch (68) corresponding to the maximum current strength and likewise extends over several treatment times and break times (11, 12).

4. Treatment device according to claim 1, **characterised in that** a plurality of base pulses (10, 37) rising like a ramp form respectively pulse packets (44), the current amplitude of which follows a rectangular function.

5. Treatment device according to claim 1, **characterised in that** the frequency of the base pulses (10, 37) in Hertz is at least a frequency of the Fibonacci sequence 1, 2, 3, 5, 8, 13, 21, 34, 55, etc.

6. Magnetic field applicator according to one of claims 1 to 5, **characterised in that** the frequency in Hertz of the pulse packets (44) formed from the base pulses (10, 37) and consisting of treatment times (11) and break times (12) corresponds at least to a frequency of the Fibonacci sequence 1, 2, 3, 5, 8, 13, 21, 34, 55, etc.

7. Treatment device according to one of claims 5 or 6, **characterised in that** the frequency in Hertz of the larger pulse packets (45, 49) formed from the pulse packets (44) and consisting of treatment times (11) and break times (12) corresponds at least to a frequency of the Fibonacci sequence 1, 2, 3, 5, 8, 13, 21, 34, 55, etc.

8. Treatment device according to one of claims 1 to 7, **characterised in that** the frequency in Hertz of the successive base pulses (10, 37) and/or the frequency of the successive pulse packets (44) and/or the frequency of the larger pulse packets (45, 49) formed from the pulse packets (44) is variable over the entire application time of the magnetic field applicator (1, 2, 5) and correspond at least to one or more of the frequencies of the Fibonacci sequence 1, 2, 3, 5, 8, 13, 21, 34, 55, etc.

9. Treatment device according to one of claims 1 to 8, **characterised in that** the frequency in Hertz of the successive treatment times (11) and/or the break times (12) is variable over the entire application time of the magnetic field applicator (1, 2, 5) and correspond at least to one or more of the frequencies of the Fibonacci sequence 1, 2, 3, 5, 8, 13, 21, 34, 55, etc.

10. Treatment device according to one of claims 1 to 9, **characterised in that** the coil current acts in periods (9) of up to 360 microseconds, and there is break between each of the periods (9).

11. Treatment device according to one of claims 1 to 10, **characterised in that** the magnetic field applicator has at least one treatment coil (5, 5a, 5b) with a magnetic core (51-54) which are installed in a treatment device (1) and generate a magnetic field emerging from a plane (2, 3, 4) of the treatment device (1).

12. Treatment device according to claim 11, **characterised in that** the magnetic field emerging from the plane (2, 3, 4) of the treatment device (1) has a spatial volume and fills a plateau surface (69) in x direction and y direction of the plane (2, 3, 4) with homogeneous magnetic flux density.

13. Treatment device according to claim 1, **characterised in that** the at least one magnetic field applicator (5, 5a, 5b) is arranged to be displaceable in a back-rest (2), seat (3) or a foot-rest, wherein its position is displayed on a scale (72) or on a display (29) of the treatment device (1).

14. Treatment device according to one of claims 1 to 13, **characterised in that** to activate and control the treatment device (1), a personalised chip card (74) is provided, on which individualised data, settings and parameters of the patient are stored for putting into operation and individualised control of the treatment device (1).

**Revendications**

1. Appareil de traitement avec un applicateur de champ magnétique (1, 55) qui est apte à générer une allure de signal en forme de rampe des courants de bobine (10, 37, 44, 49) utilisés ; l'allure de signal étant composée d'impulsions de base (10, 37) basse fréquence du courant de bobine à amplitude augmentant en forme de rampe qui agissent pendant un temps de traitement défini (11) qui font partie de paquets d'impulsion (44) composés des impulsions de base (10, 37) dont la courbe enveloppante (17, 17a, 17b) décrite par les amplitudes des impulsions de base (10, 37) est elle aussi en forme de rampe, dans lequel la courbe enveloppante (17,a, 17b, 17c) des amplitudes des impulsions de base (10, 37) forme une branche de courbe montante (57) partant de la proximité de zéro, qui monte jusqu'au milieu d'un temps de traitement (11), **caractérisé en ce que** la courbe enveloppante (17,a, 17b, 17c) des amplitudes des impulsions de base (10, 37) forme du milieu jusqu'à la fin du temps de traitement (11) une branche de courbe constante (58) correspondant à l'intensité de courant maximale, et **en ce que** l'appareil de traitement comporte des dispositifs pour la luminothérapie (35) et/ou l'oxygénothérapie et/ou pour la mesure de force (73) du patient, dans lequel les valeurs de mesure de force sont stockées et affichées sur l'affichage (29) de l'appareil de traitement (1).

2. Appareil de traitement selon la revendication 1, **caractérisé en ce qu'**une multiplicité d'impulsions de base (10, 37) montant en forme de rampe forme respectivement des paquets d'impulsions (44) dont les amplitudes de courant suivent une courbe enveloppante montante (20).

3. Appareil de traitement selon la revendication 2, **caractérisé en ce que** la courbe enveloppante montante (20) se compose d'une première branche de courbe (67) montante qui s'étend sur plusieurs temps de traitement et de pause (11, 12) et qui se prolonge par une autre branche de courbe (68) constante correspondant à l'intensité de courant maximale, qui s'étend elle aussi sur plusieurs durées de traitement et de pause (11, 12).

4. Appareil de traitement selon la revendication 1, **caractérisé en ce qu'**une multiplicité d'impulsions de base (10, 37) montant en forme de rampe forme respectivement des paquets d'impulsions (44) dont l'amplitude de courant suit une fonction rectangulaire.

5. Appareil de traitement selon la revendication 1, **caractérisé en ce que** la fréquence des impulsions de base (10, 37) en Hertz est au moins une fréquence de la suite de Fibonacci 1, 2, 3, 5, 8, 13, 21, 34, 55, etc.

6. Applicateur de champ magnétique selon l'une des revendications 1 à 5, **caractérisé en ce que** la fréquence en Hertz des paquets d'impulsions (44) composés des impulsions de base (10, 37), de temps de traitement (11) et de temps de pause (12) correspond au moins à une fréquence de la suite de Fibonacci 1, 2, 3, 5, 8, 13, 21, 34, 55, etc.

7. Appareil de traitement selon l'une des revendications 5 ou 6, **caractérisé en ce que** la fréquence en Hertz des paquets d'impulsions plus grands (45, 49) composés des paquets d'impulsions (44), de temps de traitement (11) et de temps de pause (12) correspond au moins à une fréquence de la suite de Fibonacci 1, 2, 3, 5, 8, 13, 21, 34, 55, etc.

8. Appareil de traitement selon l'une des revendications 1 à 7, **caractérisé en ce que** la fréquence en Hertz des impulsions de base (10, 37) successives et/ou la fréquence des paquets d'impulsions (44) successifs et/ou la fréquence des paquets d'impulsions plus grands (45, 49) formés des paquets d'impulsions (44) est variable sur tout le temps d'application de l'applicateur de champ magnétique (1, 2, 5) et correspond au moins à une ou plusieurs fréquences de la suite de Fibonacci 1, 2, 3, 5, 8, 13, 21, 34, 55, etc.

9. Appareil de traitement selon l'une des revendications 1 à 8, **caractérisé en ce que** la fréquence en Hertz des temps de traitement (11) successifs et ou des temps de pause (12) est variable sur tout le temps d'application de l'applicateur de champ magnétique (1, 2, 5) et correspond au moins à une ou plusieurs fréquences de la suite de Fibonacci 1, 2, 3, 5, 8, 13, 21, 34, 55, etc.

10. Appareil de traitement selon l'une des revendications 1 à 9, **caractérisé en ce que** le courant de bobine agit dans des périodes (9) allant jusqu'à 360 microsecondes et entre les périodes (9) une pause est démarrée respectivement.

11. Appareil de traitement selon l'une des revendications 1 à 10, **caractérisé en ce que** l'applicateur de champ magnétique comporte au moins une bobine de traitement (5, 5a, 5b) avec un noyau d'aimant (51-54) qui sont intégrés dans un appareil de traitement (1) et génère un champ magnétique sortant d'un plan (2, 3, 4) de l'appareil de traitement (1).

**12.** Appareil de traitement selon la revendication 11, **caractérisé en ce que** le champ magnétique sortant du plan (2, 3, 4) de l'appareil de traitement (1) comporte un volume spatial et remplit une surface plateau (69) dans les directions x et y du plan (2, 3, 4) avec une densité de flux magnétique homogène.

**13.** Appareil de traitement selon la revendication 1, **caractérisé en ce que** le au moins un applicateur de champ magnétique (5, 5a, 5b) est disposé mobile dans un dossier (2), une surface d'assise (3) ou un élément de pied, dans lequel la position dudit applicateur est affichée sur une échelle (72) ou sur un affichage (29) de l'appareil de traitement (1).

**14.** Appareil de traitement selon l'une des revendications 1 à 13, **caractérisé en ce qu'il** est prévu pour l'activation et la commande de l'appareil de traitement (1) une carte à puce personnalisée (74) sur laquelle sont stockés des données, réglages et paramètres individuels du patient pour la mise en marche et la commande individuelle de l'appareil de traitement (1).

**Figur 1**: Verhältnis der Speicherkapazitäten (Blutvolumina) C des venösen und arteriellen Systems

Venolen
( 4 % )

Venen
(3 % )

Arterien
( 19 % )

Arteriolen
( 47 % )

Kapillaren
( 27 % )

Widerstand

**Figur 2**: Verteilung von Blutvolumen und Blut-Strömungswiderstand im Körpergefäßsystem

Kapillaren
( 7 % )

Arteriolen
( 3 % )

Venolen
( 12 % )

Venen
( 63 % )

Arterien
( 15 % )

Blutvolumina

**Figur 3**: Ruheblutdruckwerte in Abhängigkeit vom Lebensalter sowie daraus abgeleiteter Normalbereich für die Verhältniszahl RPW1.

Figur 4 Blutdruckprofile in Abhängigkeit von der Uhrzeit.

Figur 5: Aus Figur 4 abgeleitete Verhältniszahl RPW₁ und Stundenmittelwerte des syst. u. diast. Blutdrucks

**Figur 6:** Anzahl von Verzweigungen in den entsprechenden Verzweigungsebenen

**Figur 7**: Spektrum eines vertikalen elektrischen Feldes natürlicher Signale einschließlich der charakteristischen *Schumann*-Resonanzen

Fig. 8

Spulenstrom [A]  10

100%
20%

7 Stufe 2
8 Stufe 1

9

0        380μs        20.000 μs    20.360 μs    t

9

Stand der Technik
Fig. 9

I
100%
20%

10    10    10
13

20ms    8s    11    12    20ms    8s    11

4s

Stand der Technik
Fig. 10

I
100%
20%

14    14    14
10

0    t

8s  4s  8s  4s  8s  4s  8s  ...
11  12  11  12  11

Stand der Technik
Fig. 11

EP 3 513 836 B1

Fig. 12

EP 3 513 836 B1

Fig. 13

Fig. 14

Fig. 15

+U, +I

+Û        +Î

I(t)    Thyristor
Gate Signal U(t)

Strom durch        21
Thyristor

0

-Û        Strom durch
Freilaufdiode

-Î

| 80µs | 80µs | 80µs | 80µs |

320µs

19.680µs        320µs

t

## Fig. 17

-U, -I        I(t)    U(t)

23        T        Gate

D        22

| +Û = +1450V | $t_{90°}$ = 80µs |
| -Û = -1450V | $t_{360°}$= 320µs |
| +Î = +1500A | $f_{360°}$= 3.125Hz |
| -Î = -1500A | $T_{RepRate}$ = 20ms(19.680µs) |
|  | $f_{RepRate}$ = 50Hz |

- Diesen Zustand (RepRate 50Hz) 10h
Dauerbetrieb pro Tag
- Umgebungstemperatur typ 38°, max. 45°C
- Thyristor/Freilaufdiode auf Kühlblech (siehe
Foto)
- Freilaufdiode muss schnell sein ($t_{rr}$<< 1µs)

## Fig. 16

## Fig. 18

# Cellvital Salut

Fig. 19

M. Kiesl 17.07.2017

EP 3 513 836 B1

EP 3 513 836 B1

Fig. 20

Fig. 21

38

a)

I

37

0    4,5  5        9,5  10    t

## Fig. 22

Trigger

4,75  5    t

## Fig. 23

b)

44

38  38

39

0    45

## Fig. 24

43  46

c)

45

0    44    136.5  333.5    t

47

## Fig. 25

48a — I    II — 48b    IX — 48c    I    II

d) ⊓⊓⊓    ⊓⊓⊓    ⊓⊓⊓    ⊓⊓⊓    ⊓⊓⊓

49    49

## Fig. 26

f (Frequens)
H$_2$

21,53

13,31

8,225

t$_1$    t$_2$    t$_3$    t$_4$    t$_5$    t

## Fig. 27

38 ← FB-Frequenzreihe A

38,38,38

38,38,38

44 ← FB-Frequenzreihe B

44

44

49 ← FB-Frequenzreihe C

Fig. 28

Fig. 29

Fig. 30

Fig. 31

Fig. 32

42

Fig. 33

Fig. 34

Fig. 35

Fig. 36

Fig. 37

Fig. 38

Fig. 39

Fig. 40

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0594655 B1
- EP 0594644 B1
- WO 2004087255 A1
- WO 2016074726 A1
- DE 202016008331 U1

**In der Beschreibung aufgeführte Nicht-Patentliteratur**

- **TOOMEY, J. ; C. POLK.** Research on Extremely Low Frequency Propagation with Particular Emphasis on Schumann Resonance and Related Phenomena. University of Rhode Island, 01. April 1970, 4950
- **S. S. SHAPIRO ; M. B. WILK.** An Analysis of Variance Test for Normality (Complete Samples). *Biometrica,* vol. 52 (3-4), 591-611